# EUROPEAN PATENT SPECIFICATION

(11) **EP 2 773 957 B1**
(45) Date of publication and mention of the grant of the patent: **29.11.2017**
(21) Application number: 12846452.6
(22) Date of filing: 29.10.2012
(51) Int. Cl.: G01N 33/53, A61K 39/395

(54) **USE OF AN ANTI-ALPHA-SYNUCLEIN ANTIBODY TO DIAGNOSE AN ELEVATED LEVEL OF ALPHA-SYNUCLEIN IN THE BRAIN**
VERWENDUNG EINES ANTI-ALPHA-SYNUCLEIN-ANTIKÖRPERS ZUR DIAGNOSE EINER HOHEN KONZENTRATION VON ALPHA- SYNUCLEIN IM GEHIRN
UTILISATION D'UN ANTICORPS ANTI-ALPHA-SYNUCLÉINE POUR DIAGNOSTIQUER UN NIVEAU ÉLEVÉ D'ALPHA-SYNUCLÉINE DANS LE CERVEAU

(30) Priority: 02.11.2011 US 201161554924 P
(43) Date of publication of application: 10.09.2014
(73) Proprietor: Biogen International Neuroscience GmbH, 6300 Zug (CH)
(72) Inventor: WEIHOFEN, Andreas, 8037 Zurich (CH); ENGBER, Thomas, Boston, MA 02118 (US); GRIMM, Jan, 8600 Dübendorf (CH)
(74) Representative: Müller Fottner Steinecke
(86) International application number: PCT/US2012/062430
(87) International publication number: WO 2013/066818

(56) References cited:
- WO-A1-2010/069603
- US-A1- 2008 300 204
- US-A1- 2011 052 498
- MOLLENHAUER BRIT ET AL: "Quantification of alpha-synuclein in cerebrospinal fluid as a biomarker candidate: review of the literature and considerations for future studies", BIOMARKERS IN MEDICINE JAN 2014, BIOMARKERS IN MEDICINE, vol. 4, no. 5, 1 October 2010 (2010-10-01) , pages 683-699, XP009152858, ISSN: 1752-0363, DOI: 10.2217/BMM.10.90

### SEQUENCE LISTING

<110> BIOGEN IDEC INTERNATIONAL NEUROSCIENCE GMBH
   WEIHOFEN, Andreas
   ENGBER, Thomas
   GRIMM, Jan
<120> USE OF AN ANTI-ALPHA-SYNUCLEIN ANTIBODY TO DIAGNOSE AN ELEVATED LEVEL OF ALPHA-SYNUCLEIN IN THE BRAIN
<130> 2159.356PC01
<140> To be assigned
   <141> Herewith
<150> 61/554,924
   <151> 11-02-2011
<160> 9
<170> PatentIn version 3.5
<210> 1
   <211> 140
   <212> PRT
   <213> Homo sapiens
<220>
   <223> Alpha-synuclein
<400> 1
<210> 2
   <211> 113
   <212> PRT
   <213> Artificial Sequence
<220>
   <223> heavy chain variable region (VH)
<400> 2
   Glu Val Gln Leu Val Gln Ser Gly Gly Gly Leu Val Glu Pro Gly Gly
   1               5                   10                  15
   Ser Leu Arg Leu Ser Cys Ala Val Ser Gly Phe Asp Phe Glu Lys Ala
               20                  25                  30
   Trp Met Ser Trp Val Arg Gln Ala Pro Gly Gln Gly Leu Gln Trp Val
           35                  40                  45
   Ala Arg Ile Lys Ser Thr Ala Asp Gly Gly Thr Thr Ser Tyr Ala Ala
       50                  55                  60
   Pro Val Glu Gly Arg Phe Ile Ile Ser Arg Asp Asp Ser Arg Asn Met
   65                  70                  75                  80
   Leu Tyr Leu Gln Met Asn Ser Leu Lys Thr Glu Asp Thr Ala Val Tyr
                   85                  90                  95
   Tyr Cys Thr Ser Ala His Trp Gly Gln Gly Thr Leu Val Thr Val Ser
               100                 105                 110
   Ser
<210> 3
   <211> 108
   <212> PRT
   <213> Artificial Sequence
<220>
   <223> light chain variable region (VL)
<400> 3
   Gln Ser Val Leu Thr Gln Pro Pro Ser Val Ser Val Ser Pro Gly Gln
   1               5                   10                  15
   Thr Ala Arg Ile Thr Cys Ser Gly Glu Ala Leu Pro Met Gln Phe Ala
   20 25 30
   His Trp Tyr Gln Gln Arg Pro Gly Lys Ala Pro Val Ile Val Val Tyr
   35 40 45
   Lys Asp Ser Glu Arg Pro Ser Gly Val Pro Glu Arg Phe Ser Gly Ser
   50 55 60
   Ser Ser Gly Thr Thr Ala Thr Leu Thr Ile Thr Gly Val Gln Ala Glu
   65 70 75 80
   Asp Glu Ala Asp Tyr Tyr Cys Gln Ser Pro Asp Ser Thr Asn Thr Tyr
   85 90 95
   Glu Val Phe Gly Gly Gly Thr Lys Leu Thr Val Leu
   100 105
<210> 4
   <211> 5
   <212> PRT
   <213> Artificial Sequence
<220>
   <223> complementarity determining region-1 (VHCDR1)
<400> 4
   Lys Ala Trp Met Ser
   1 5
<210> 5
   <211> 19
   <212> PRT
   <213> Artificial Sequence
<220>
   <223> complementarity determining region-2 (VHCDR2)
<400> 5
   Arg Ile Lys Ser Thr Ala Asp Gly Gly Thr Thr Ser Tyr Ala Ala Pro
   1 5 10 15
   Val Glu Gly
<210> 6
   <211> 2
   <212> PRT
   <213> Artificial Sequence
<220>
   <223> complementarity determining region-3 (VHCDR3)
<400> 6
   Ala His
   1
<210> 7
   <211> 11
   <212> PRT
   <213> Artificial Sequence
<220>
   <223> complementarity determining region-1 (VLCDR1)
<400> 7
   Ser Gly Glu Ala Leu Pro Met Gln Phe Ala His
   1               5                   10
<210> 8
   <211> 7
   <212> PRT
   <213> Artificial Sequence
<220>
   <223> complementarity determining region-2 (VLCDR2)
<400> 8
   Lys Asp Ser Glu Arg Pro Ser
   1               5
<210> 9
   <211> 11
   <212> PRT
   <213> Artificial Sequence
<220>
   <223> complementarity determining region-3 (VLCDR3)
<400> 9
   Gln Ser Pro Asp Ser Thr Asn Thr Tyr Glu Val
   1               5                   10

## Description

### BACKGROUND

### Field of the Disclosure

This disclosure relates to the use of anti-α-synuclein antibody to diagnose an elevated level of α-synuclein in the brain. Specifically, the disclosure relates to the method of assessing the levels of α-synuclein in blood plasma or cerebrospinal fluid (CSF) following administration to the test subject of an anti-α-synuclein antibody or antigen-binding fragment thereof, which can bind α-synuclein with sufficient activity to alter the net efflux of α-synuclein from brain to blood or brain to CSF.

### Background of the Disclosure

The mammalian brain is separated from blood by the blood-brain barrier (BBB) localized to the brain capillaries and pia-subarachnoid membranes and the blood-cerebrospinal fluid (CSF) barrier localized to the choriod plexi. α-synuclein is relatively abundant in the brain under non-pathological conditions. It is a natively unfolded protein present mostly in the cytosol. It plays an essential role in synaptic transmission and synaptic plasticity by augmenting transmitter release from the presynaptic terminal. (Liu et al., EMBO J 23:4506-4516 (2004)). Mutations in α-synuclein are associated with rare familial cases of early-onset Parkinson's disease, and conversion of α-synuclein from its soluble into the aggregated insoluble form is one of key events in the pathogenesis of neurodegenerative disorders, such as Parkinson's disease, dementia with Lewy bodies (DLB), and several other neurodegenerative illnesses. (Dawson et al., Science 302:819-822 (2003), Bennett et al., Pharmacol Ther. 105:311-331 (2005), George, JM., Genome Biol. 3(1): reviews3002.1-reviews3002.6 (2002)). Additionally, both monomeric and oligomeric α-synuclein have been found in the cerebrospinal fluid (CSF) and serum of Parkinson's disease patients, as apparently α-synuclein and even its aggregated species can cross the blood-brain barrier. (El-Agnaf et al., FASEB J. 20:419-425 (2006), Tokuda et al., Biochem Biophys Res Commun. 349:162-166 (2006), Lee et al., J Neural Transm. 113:1435-1439 (2006), Mollenhauer et al., Exp Neurol. 213:315-325 (2008), El-Agnaf et al., FASEB J. 17:1945-1947 (2003), Li et al., Exp Neurol 204: 583-588 (2007).

Immunization studies in mouse models of Parkinson's disease show that mouse monoclonal antibodies against α-synuclein can reduce accumulation of intracellular α-synuclein aggregates (Masliah et al., Neuron, 46: 857-868 (2005); Masliah et al., PLoS One, 6(4): e19338 (2011) supporting the idea that antibodies that neutralize the neurotoxic aggregates without interfering with beneficial functions of monomeric α-synuclein can be useful therapeutics. However, the therapeutic and diagnostic utility of murine based antibodies in human is hampered by the human anti-mouse antibody (HAMA) response in view of their non-human origin.

Accordingly, there is a need to develop a method for assessing patients for elevated levels of α-synuclein in the brain.

### BRIEF SUMMARY

The solution of the problem underlying the present invention is provided by the embodiments characterized in the claims.

One embodiment is directed to a method of diagnosing an elevated level of α-synuclein in the brain of a test subject comprising: (a) assaying the level of α-synuclein in a blood plasma sample obtained from the test subject at a specified interval following peripheral administration to the test subject of an anti-α-synuclein antibody or antigen-binding fragment thereof, wherein the antibody or fragment thereof can bind α-synuclein with sufficient affinity to alter the net efflux of α-synuclein from brain to blood; (b) comparing the assayed level of the α-synuclein in the test subject to a reference standard; wherein the difference or similarity between the level of α-synuclein in the plasma sample and the reference standard correlates with the level of α-synuclein in the brain of the test subject. In accordance with the present invention the test subject is a human subject.

Also disclosed is a method of diagnosing an elevated level of α-synuclein in the brain of a test subject comprising: (a) providing an anti-α-synuclein antibody or antigen-binding fragment thereof, wherein the antibody or fragment thereof can bind α-synuclein with sufficient affinity to alter the net efflux of α-synuclein from brain to blood; (b) directing a healthcare provider to peripherally administer the antibody to the test subject and obtain a blood plasma sample from the subject at a specified time interval following administration; (c) assaying the level of α-synuclein in the blood plasma sample; (d) comparing the assayed level of α-synuclein in the test subject to a reference standard; wherein the difference or similarity between the level of the α-synuclein in the plasma sample and the reference standard correlates with the level of α-synuclein in the brain of the test subject.

Further disclosed is a method of diagnosing an elevated level of α-synuclein in the brain of a test subject comprising: (a) peripherally administering an anti-α-synuclein antibody or antigen-binding fragment thereof to the test subject, wherein the antibody or fragment thereof can bind α-synuclein with sufficient affinity to alter the net efflux of the α-synuclein from brain to blood; (b) obtaining a blood plasma sample from the test subject at a specified time interval following administration, and submitting the sample for determination of the level of the α-synuclein; (c) comparing the level of the α-synuclein in blood plasma sample to a reference standard; wherein the difference or similarity between the level of the α-synuclein in the plasma sample and the reference standard correlates with level of the α-synuclein in the brain of the test subject.

Further disclosed is the method as described herein, further comprising comparing the level of the α-synuclein in the plasma sample to a plasma sample obtained from the test subject prior to administration of the anti-α-synuclein antibody or antigen-binding fragment thereof.

In specific embodiments, the reference standard in the above-described method comprises measured levels of α-synuclein in one or more control subjects, wherein the control subjects include normal healthy individuals, and individuals with synucleinopathies of varying severity.

Further disclosed is a method of tracking the α-synuclein level in the brain of a subject being treated for a synucleinopathic disease, comprising assaying the level of α-synuclein in the subject's blood plasma at a specified time following peripheral administration of an anti-α-synuclein antibody or antigen-binding fragment thereof, wherein the antibody or fragment thereof can bind α-synuclein with sufficient affinity to alter the net efflux of the α-synuclein from brain to blood; and wherein the α-synuclein level in the subject's blood plasma correlates with the level in the subject's brain. In specific embodiments, the above-described method, further comprises assaying the level of α-synuclein in the subject's blood plasma at a specified time following additional peripheral administrations of the anti-α-synuclein antibody or antigen-binding fragment thereof, thereby plotting the change in the α-synuclein level in the subject's brain over time.

Some embodiments include the method as described herein, where the method is directed to diagnosing an elevated level of α-synuclein in the brain of a test subject by assaying the level of α-synuclein in a CSF sample obtained from the test subject at a specified time intervals following administrations of an anti-α-synuclein antibody or antigen-binding fragment thereof, wherein the antibody or fragment thereof can bind α-synuclein with sufficient affinity to alter the net efflux of the α-synuclein from brain to CSF, and wherein the difference or similarity between the level of the α-synuclein in the CSF sample and the reference standard correlates with level of the α-synuclein in the brain of the test subject. Some embodiments include the method as described herein, further comprising comparing the level of the α-synuclein in the CSF sample to a CSF sample obtained from the test subject prior to administration of the anti-α-synuclein antibody or antigen-binding fragment thereof.

Some embodiments include the method of tracking the α-synuclein level in the brain of a subject being treated for a synucleinopathic disease, comprising assaying the level of α-synuclein in the subject's CSF sample at a specified time following peripheral administration of an anti-α-synuclein antibody or antigen-binding fragment thereof, wherein the antibody or fragment thereof can bind α-synuclein with sufficient affinity to alter the net efflux of the α-synuclein from brain to CSF; and wherein the α-synuclein level in the subject's CSF correlates with the level in the subject's brain.

Some embodiments include the method as described herein, wherein the antibody or antigen-binding fragment thereof specifically binds to the same α-synuclein epitope a reference antibody comprising a VH and a VL, wherein the VH comprises SEQ ID NO: 2 and the VL comprises SEQ ID NO: 3. In some embodiments, the antibody or antigen-binding fragment thereof competitively inhibits a reference antibody comprising a VH and a VL, wherein the VH comprises SEQ ID NO: 2 and the VL comprises SEQ ID NO: 3 from binding to α-synuclein.

Further provided is the method as described herein, wherein the antibody or antigen-binding fragment thereof comprises a heavy chain variable region (VH) and a light chain variable region (VL), wherein the VH comprises a complementarity determining region-1 (VHCDR1) amino acid sequence of SEQ ID NO: 4.

Also provided is the method as described herein, wherein the antibody or antigen-binding fragment thereof comprises a VH and a VL, wherein the VH comprises a complementarity determining region-2 (VHCDR2) amino acid sequence of SEQ ID NO: 5.

Some embodiments include the method as described herein, wherein the antibody or antigen-binding fragment thereof comprises a VH and a VL, wherein the VH comprises a complementarity determining region-3 (VHCDR3) amino acid sequence of SEQ ID NO: 6.

Also provided is the method as described herein, wherein the antibody or antigen-binding fragment thereof comprises a VH and a VL, wherein the VL comprises a complementarity determining region-1 (VLCDR1) amino acid sequence of SEQ ID NO: 7.

Some embodiments include the method as described herein, wherein the antibody or antigen-binding fragment thereof comprises a VH and a VL, wherein the VL comprises a complementarity determining region-2 (VLCDR2) amino acid sequence of SEQ ID NO: 8.

Also disclosed is the method as described herein, wherein the antibody or antigen-binding fragment thereof comprises a VH and a VL, wherein the VL comprises a complementarity determining region-3 (VLCDR3) amino acid sequence of SEQ ID NO: 9.

Further provided is the method as described herein, wherein the antibody or antigen-binding fragment thereof comprises wherein the antibody or antigen-binding fragment thereof comprises a VH and a VL, wherein the VH comprises VHCDR1, VHCDR2, and VHCDR3 amino acid sequences of SEQ ID NOs: 4, 5, 6.

Also disclosed is the method as described herein, wherein the antibody or antigen-binding fragment thereof comprises a VH and a VL, wherein the VL comprises VLCDR1, VLCDR2, and VLCDR3 amino acid sequences of SEQ ID NOs: 7, 8, 9.

Some embodiments include the method as described herein, wherein the antibody or antigen-binding fragment thereof comprises a VH and a VL, wherein the VH comprises VHCDR1, VHCDR2, and VHCDR3 amino acid sequences of SEQ ID NOs: 5, 6, 7, and the VL, comprises VLCDR1, VLCDR2, and VLCDR3 amino acid sequences of SEQ ID NOs: 7, 8, 9.

Some embodiments include the method as described herein, wherein the antibody or antigen binding fragment thereof comprises a VH amino acid sequence of SEQ ID NO: 2 and a VL amino acid sequence of SEQ ID NO: 3.

Also disclosed is the method as described herein, wherein the antibody or antigen binding fragment thereof is a single chain Fv fragment (scFv), an F(ab') fragment, an F(ab) fragment, or an F(ab')₂ fragment.

Some embodiments include the method as described herein, wherein the administering is by intravenous injection of the antibody. In one embodiment the antibody is human.

Other embodiments include the method as described herein, wherein the specified time interval is less than a week, or less than or equal to 24 hours, or less than or equal to 3 hours.

Certain embodiments include the method as described herein, wherein the synucleinopathic disease is selected from the group consisting of Parkinson's disease (PD), Parkinson's disease dementia (PDD), dementia with Lewy bodies (DLB), the Lewy body variant of Alzheimer's disease (LBVAD), multiple systems atrophy (MSA), pure autonomic failure (PAF), neurodegeneration with brain iron accumulation type-1 (NBIA-I), Alzheimer's disease, Pick disease, juvenile-onset generalized neuroaxonal dystrophy (Hallervorden-Spatz disease), amyotrophic lateral sclerosis, traumatic brain injury and Down syndrome.

### BRIEF DESCRIPTION OF THE DRAWINGS/FIGURES

Figure 1 (A-B): Dose dependent human α-synuclein plasma spike upon 12F4 antibody administration in transgenic mice overexpressing human α-synuclein.
Figure 2: Time course of human α-synuclein plasma spike and plasma 12F4 antibody concentrations.
Figure 3 (A-C): Acute, high dose 12F4 antibody treatment of transgenic mice overexpressing human α-synuclein reduces brain human α-synuclein levels.
Figure 4 (A-C): Human α-synuclein plasma levels significantly reflect brain α-synuclein levels after 12F4 antibody injection.
Figure 5 (A-C): Plasma human α-synuclein (A) and chimeric 12F4 antibody levels (B) were determined by ELISA. (C) There was a significant correlation between plasma and brain α-synuclein levels after chronic treatment for six month with chimeric 12F4 antibody of transgenic mice overexpressing human α-synuclein.
Figure 6: α-synuclein cerebrospinal fluid (CSF) spike and CSF/serum 124F ratio around 0.1% upon 12F4 administration in cynomolgus monkeys.
Figure 7: *In vivo* microdialysis in transgenic α-synuclein mice shows drop of brain interstitial fluid (ISF) α-synuclein upon 12F4 administration.

### DETAILED DESCRIPTION

### I. DEFINITIONS

It is to be noted that the term "a" or "an" entity refers to one or more of that entity; for example, "an anti-α-synuclein antibody," is understood to represent one or more antibodies which specifically bind to α-synuclein. As such, the terms "a" (or "an"), "one or more," and "at least one" can be used interchangeably herein.

As used herein, the terms "synucleinopathic diseases" or "synucleinopathies" are a diverse group of neurodegenerative disorders that share a common pathologic lesion composed of aggregates of insoluble α-synuclein protein in selectively vulnerable populations of neurons and glia. These disorders include Parkinson's disease (PD), Parkinson's disease dementia (PDD), dementia with Lewy bodies (DLB), the Lewy body variant of Alzheimer's disease (LBVAD), multiple systems atrophy (MSA), pure autonomic failure (PAF), neurodegeneration with brain iron accumulation type-1 (NBIA-I), Alzheimer's disease, Pick disease, juvenile-onset generalized neuroaxonal dystrophy (Hallervorden-Spatz disease), amyotrophic lateral sclerosis, traumatic brain injury and Down syndrome. Clinically, they are characterized by a chronic and progressive decline in motor, cognitive, behavioral, and autonomic functions, depending on the distribution of the lesions.

Unless stated otherwise, the terms "disorder" "disease" and "illness" are used interchangeably herein.

As used herein, the terms "binding molecule" or "antigen binding molecule" refers in its broadest sense to a molecule that specifically binds an antigenic determinant. Non-limiting examples of antigen binding molecules are antibodies and fragments thereof that retain antigen-specific binding, as well as other non-antibody molecules that bind to α-synuclein including but not limited to hormones, receptors, ligands, major histocompatibility complex (MHC) molecules, chaperones such as heat shock proteins (HSPs) as well as cell-cell adhesion molecules such as members of the cadherin, intergrin, C-type lectin and immunoglobulin (Ig) superfamilies. Thus, for the sake of clarity only and without restricting the scope of the disclosure most of the following embodiments are discussed with respect to antibodies and antibody-like molecules which represent the binding molecules for the development of therapeutic and diagnostic agents. In another embodiment, a binding molecule disclosed comprises at least one heavy or light chain CDR of an antibody molecule. In another embodiment, a binding molecule disclosed comprises at least two CDRs from one or more antibody molecules. In another embodiment, a binding molecule disclosed comprises at least three CDRs from one or more antibody molecules. In another embodiment, a binding molecule as disclosed comprises at least four CDRs from one or more antibody molecules. In another embodiment, a binding molecule as disclosed comprises at least five CDRs from one or more antibody molecules. In another embodiment, a binding molecule as disclosed comprises at least six CDRs from one or more antibody molecules.

Disclosed herein is a method of diagnosing an elevated level of α-synuclein in the brain of a test subject, comprising administering to the subject an anti-α-synuclein binding molecule, *e.g,.* an antibody, or antigen-binding fragment, variant, or derivative thereof. Unless specifically referring to full-sized antibodies such as naturally occurring antibodies, the term "anti-α-synuclein antibody" encompasses full-sized antibodies as well as antigen-binding fragments, variants, analogs, or derivatives of such antibodies, *e.g*., naturally occurring antibody or immunoglobulin molecules or engineered antibody molecules or fragments that bind antigen in a manner similar to antibody molecules.

The terms "antibody" and "immunoglobulin" are used interchangeably herein. An antibody or immunoglobulin comprises at least the variable domain of a heavy chain, and normally comprises at least the variable domains of a heavy chain and a light chain. Basic immunoglobulin structures in vertebrate systems are relatively well understood. See, *e.g*., Harlow et al. (1988) Antibodies: A Laboratory Manual (2nd ed.; Cold Spring Harbor Laboratory Press).

As used herein, the term "immunoglobulin" comprises various broad classes of polypeptides that can be distinguished biochemically. Those skilled in the art will appreciate that heavy chains are classified as gamma, mu, alpha, delta, or epsilon, (γ, µ, α, δ, ε) with some subclasses among them (*e.g*., γ1-γ4). It is the nature of this chain that determines the "class" of the antibody as IgG, IgM, IgA IgG, or IgE, respectively. The immunoglobulin subclasses (isotypes) *e.g.,* IgG1, IgG2, IgG3, IgG4, IgA1, *etc.* are well characterized and are known to confer functional specialization. Modified versions of each of these classes and isotypes are readily discernable to the skilled artisan in view of the disclosure and, accordingly, are within the scope of the disclosure. All immunoglobulin classes are clearly within the scope of the disclosure. The following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Light chains are classified as either kappa or lambda (κ, λ). Each heavy chain class can be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain.

Both the light and heavy chains are divided into regions of structural and functional homology. The terms "constant" and "variable" are used functionally. In this regard, it will be appreciated that the variable domains of both the light (VL or VK) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention the numbering of the constant region domains increases as they become more distal from the antigen binding site or amino-terminus of the antibody. The N-terminal portion is a variable region and at the C-terminal portion is a constant region; the CH3 and CL domains actually comprise the carboxy-terminus of the heavy and light chain, respectively.

As indicated above, the variable region allows the antibody to selectively recognize and specifically bind epitopes on antigens. That is, the VL domain and VH domain, or subset of the complementarity determining regions (CDRs) within these variable domains, of an antibody combine to form the variable region that defines a three dimensional antigen binding site. This quaternary antibody structure forms the antigen binding site present at the end of each arm of the Y. More specifically, the antigen binding site is defined by three CDRs on each of the VH and VL chains. In some instances, e.g., certain immunoglobulin molecules derived from camelid species or engineered based on camelid immunoglobulins, a complete immunoglobulin molecule can consist of heavy chains only, with no light chains. See, *e.g.,* Hamers-Casterman et al., Nature 363:446-448 (1993).

In naturally occurring antibodies, the six "complementarity determining regions" or "CDRs" present in each antigen binding domain are short, non-contiguous sequences of amino acids that are specifically positioned to form the antigen binding domain as the antibody assumes its three dimensional configuration in an aqueous environment. The remainder of the amino acids in the antigen binding domains, referred to as "framework" regions, show less inter-molecular variability. The framework regions largely adopt a β-sheet conformation and the CDRs form loops that connect, and in some cases form part of, the β-sheet structure. Thus, framework regions act to form a scaffold that provides for positioning the CDRs in correct orientation by inter-chain, non-covalent interactions. The antigen binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable domain by one of ordinary skill in the art, since they have been precisely defined (see below).

In the case where there are two or more definitions of a term that is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al. (1983) U.S. Dept. of Health and Human Services, "Sequences of Proteins of Immunological Interest" and by Chothia and Lesk, J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues that encompass the CDRs as defined by each of the above cited references are set forth below in Table 1 as a comparison. The exact residue numbers that encompass a particular CDR will vary depending on the sequence and size of the CDR. Those skilled in the art can routinely determine which residues comprise a particular CDR given the variable region amino acid sequence of the antibody.

**Table 1. CDR Definitions¹**

| | Kabat | Chothia |
|---|---|---|
| VH CDR1 | 31-35 | 26-32 |
| VH CDR2 | 50-65 | 52-58 |
| VH CDR3 | 95-102 | 95-102 |
| VL CDR1 | 24-34 | 26-32 |
| VL CDR2 | 50-56 | 50-52 |
| VL CDR3 | 89-97 | 91-96 |

| | | |
|---|---|---|
| ¹Numbering of all CDR definitions in Table 1 is according to the numbering conventions set forth by Kabat et al. (see below). | | |

*Kabat et al.* also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al. (1983) U.S. Dept. of Health and Human Services, "Sequence of Proteins of Immunological Interest." Unless otherwise specified, references to the numbering of specific amino acid residue positions in an anti-α-synuclein antibody or antigen-binding fragment, variant, or derivative thereof of the present disclosure are according to the Kabat numbering system.

Antibodies or antigen-binding fragments, variants, or derivatives thereof of the disclosure include, but are not limited to, polyclonal, monoclonal, multispecific, human, humanized, primatized, or chimeric antibodies, single-chain antibodies, epitope-binding fragments, *e.g*., Fab, Fab' and F(ab')₂, Fd, Fvs, single-chain Fvs (scFv), disulfide-linked Fvs (sdFv), fragments comprising either a VL or VH domain, fragments produced by a Fab expression library, and anti-idiotypic (anti-Id) antibodies. ScFv molecules are known in the art and are described, *e.g*., in U.S. Pat. No. 5,892,019. Immunoglobulin or antibody molecules of the disclosure can be of any type (e.g., IgG, IgE, IgM, IgD, IgA, and IgY), class (*e.g.,* IgG1, IgG2, IgG3, IgG4, IgA1, and IgA2, etc.), or subclass of immunoglobulin molecule.

As used herein, the term "heavy chain portion" includes amino acid sequences derived from an immunoglobulin heavy chain. In certain embodiments, a polypeptide comprising a heavy chain portion comprises at least one of: a VH domain, a CH1 domain, a hinge (e.g., upper, middle, and/or lower hinge region) domain, a CH2 domain, a CH3 domain, or a variant or fragment thereof. For example, a binding polypeptide for use in the disclosure can comprise a polypeptide chain comprising a CH1 domain; a polypeptide chain comprising a CH1 domain, at least a portion of a hinge domain, and a CH2 domain; a polypeptide chain comprising a CH1 domain and a CH3 domain; a polypeptide chain comprising a CH1 domain, at least a portion of a hinge domain, and a CH3 domain, or a polypeptide chain comprising a CH1 domain, at least a portion of a hinge domain, a CH2 domain, and a CH3 domain. In another embodiment, a polypeptide of the disclosure comprises a polypeptide chain comprising a CH3 domain. Further, a binding polypeptide for use in the disclosure can lack at least a portion of a CH2 domain (e.g., all or part of a CH2 domain). As set forth above, it will be understood by one of ordinary skill in the art that these domains (e.g., the heavy chain portions) can be modified such that they vary in amino acid sequence from the naturally occurring immunoglobulin molecule.

In certain embodiments, anti-α-synuclein antibodies, or antigen-binding fragments, variants, or derivatives thereof disclosed herein, the heavy chain portions of one polypeptide chain of a multimer are identical to those on a second polypeptide chain of the multimer. Alternatively, heavy chain portion-containing monomers of the disclosure are not identical. For example, each monomer can comprise a different target binding site, forming, for example, a bispecific antibody.

The heavy chain portions of a binding molecule for use in the methods disclosed herein can be derived from different immunoglobulin molecules. For example, a heavy chain portion of a polypeptide can comprise a C_{H1} domain derived from an IgG1 molecule and a hinge region derived from an IgG3 molecule. In another example, a heavy chain portion can comprise a hinge region derived, in part, from an IgG1 molecule and, in part, from an IgG3 molecule. In another example, a heavy chain portion can comprise a chimeric hinge derived, in part, from an IgG1 molecule and, in part, from an IgG4 molecule.

As used herein, the term "light chain portion" includes amino acid sequences derived from an immunoglobulin light chain, *e.g.,* a kappa or lambda light chain. Preferably, the light chain portion comprises at least one of a VL or CL domain.

As previously indicated, the subunit structures and three dimensional configuration of the constant regions of the various immunoglobulin classes are well known. As used herein, the term "VH domain" includes the amino terminal variable domain of an immunoglobulin heavy chain and the term "CH1 domain" includes the first (most amino terminal) constant region domain of an immunoglobulin heavy chain. The CH1 domain is adjacent to the VH domain and is amino terminal to the hinge region of an immunoglobulin heavy chain molecule.

As used herein the term "CH2 domain" includes the portion of a heavy chain molecule that *extends, e.g.,* from about residue 244 to residue 360 of an antibody using conventional numbering schemes (residues 244 to 360, Kabat numbering system; and residues 231-340, EU numbering system; see Kabat *EA et al. op. cit.* The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues.

As used herein, the term "hinge region" includes the portion of a heavy chain molecule that joins the CH1 domain to the CH2 domain. This hinge region comprises approximately 25 residues and is flexible, thus allowing the two N-terminal antigen binding regions to move independently. Hinge regions can be subdivided into three distinct domains: upper, middle, and lower hinge domains (Roux et al., J. Immunol. 161:4083 (1998)).

As used herein the term "disulfide bond" includes the covalent bond formed between two sulfur atoms. The amino acid cysteine comprises a thiol group that can form a disulfide bond or bridge with a second thiol group. In most naturally occurring IgG molecules, the CH1 and CL regions are linked by a disulfide bond and the two heavy chains are linked by two disulfide bonds at positions corresponding to 239 and 242 using the Kabat numbering system (position 226 or 229, EU numbering system).

Anti-α-synuclein antibodies, or antigen-binding fragments, variants, or derivatives thereof disclosed herein can be described or specified in terms of the epitope(s) or portion(s) of an antigen, e.g., a target polypeptide disclosed herein (e.g., α-synuclein) that they recognize or specifically bind. The portion of a target polypeptide that specifically interacts with the antigen binding domain of an antibody is an "epitope," or an "antigenic determinant." A target polypeptide can comprise a single epitope, but typically comprises at least two epitopes, and can include any number of epitopes, depending on the size, conformation, and type of antigen. Furthermore, it should be noted that an "epitope" on a target polypeptide can be or can include non-polypeptide elements, e.g., an epitope can include a carbohydrate side chain.

The minimum size of a peptide or polypeptide epitope for an antibody is thought to be about four to five amino acids. Peptide or polypeptide epitopes preferably contain at least seven, more preferably at least nine and most preferably between at least about 15 to about 30 amino acids. Since a CDR can recognize an antigenic peptide or polypeptide in its tertiary form, the amino acids comprising an epitope need not be contiguous, and in some cases, can not even be on the same peptide chain. A peptide or polypeptide epitope recognized by anti-α-synuclein. antibodies of the disclosure can contain a sequence of at least 4, at least 5, at least 6, at least 7, more preferably at least 8, at least 9, at least 10, at least 15, at least 20, at least 25, or between about 15 to about 30 contiguous or non-contiguous amino acids of α-synuclein.

By "specifically binds," it is generally meant that an antibody binds to an epitope via its antigen binding domain, and that the binding entails some complementarity between the antigen binding domain and the epitope. According to this definition, an antibody is said to "specifically bind" to an epitope when it binds to that epitope, via its antigen binding domain more readily than it would bind to a random, unrelated epitope. The term "specificity" is used herein to qualify the relative affinity by which a certain antibody binds to a certain epitope. For example, antibody "A" can be deemed to have a higher specificity for a given epitope than antibody "B," or antibody "A" can be said to bind to epitope "C" with a higher specificity than it has for related epitope "D."

By "preferentially binds," it is meant that the antibody specifically binds to an epitope more readily than it would bind to a related, similar, homologous, or analogous epitope. Thus, an antibody that "preferentially binds" to a given epitope would more likely bind to that epitope than to a related epitope, even though such an antibody can cross-react with the related epitope.

By way of non-limiting example, an antibody can be considered to bind a first epitope preferentially if it binds said first epitope with a dissociation constant (K_{D}) that is less than the antibody's K_{D} for the second epitope. In another non-limiting example, an antibody can be considered to bind a first antigen preferentially if it binds the first epitope with an affinity that is at least one order of magnitude less than the antibody's K_{D} for the second epitope. In another non-limiting example, an antibody can be considered to bind a first epitope preferentially if it binds the first epitope with an affinity that is at least two orders of magnitude less than the antibody's K_{D} for the second epitope.

In another non-limiting example, an antibody can be considered to bind a first epitope preferentially if it binds the first epitope with an off rate (k(off)) that is less than the antibody's k(off) for the second epitope. In another non-limiting example, an antibody can be considered to bind a first epitope preferentially if it binds the first epitope with an affinity that is at least one order of magnitude less than the antibody's k(off) for the second epitope. In another non-limiting example, an antibody can be considered to bind a first epitope preferentially if it binds the first epitope with an affinity that is at least two orders of magnitude less than the antibody's k(off) for the second epitope.

An antibody or antigen-binding fragment, variant, or derivative disclosed herein can be said to bind a target polypeptide disclosed herein (*e.g*., human α-synuclein) or a fragment or variant thereof with an off rate (k(off)) of less than or equal to 5 X 10⁻² sec⁻¹, 10⁻² sec⁻¹, 5 X 10⁻³ sec⁻¹ or 10⁻³ sec⁻¹. More preferably, an antibody of the disclosure can be said to bind a target polypeptide disclosed herein (e.g., human α-synuclein) or a fragment or variant thereof with an off rate (k(off)) less than or equal to 5 X 10⁻⁴ sec⁻¹, 10⁻⁴ sec⁻¹, 5 X 10⁻⁵ sec⁻¹, or 10⁻⁵ sec⁻¹, 5 X 10⁻⁶ sec⁻¹, 10⁻⁶ sec⁻¹, 5 X 10⁻⁷ sec⁻¹ or 10⁻⁷ sec⁻¹.

An antibody or antigen-binding fragment, variant, or derivative disclosed herein can be said to bind a target polypeptide disclosed herein (*e.g*., human α-synuclein) or a fragment or variant thereof with an on rate (k(on)) of greater than or equal to 10³ M⁻¹ sec⁻¹, 5 X 10³ M⁻¹ sec⁻¹, 10⁴ M⁻¹ sec⁻¹ or 5 X 10⁴ M⁻¹ sec⁻¹. More preferably, an antibody of the disclosure can be said to bind a target polypeptide disclosed herein (e.g., human α-synuclein) or a fragment or variant thereof with an on rate (k(on)) greater than or equal to 10⁵ M⁻¹ sec⁻¹, 5 X 10⁵ M⁻¹ sec⁻¹, 10⁶ M⁻¹ sec⁻¹, or 5 X 10⁶ M⁻¹ sec⁻¹ or 10⁷ M⁻¹ sec⁻¹.

An antibody is said to competitively inhibit binding of a reference antibody to a given epitope if it preferentially binds to that epitope to the extent that it blocks, to some degree, binding of the reference antibody to the epitope. Competitive inhibition can be determined by any method known in the art, for example, competition ELISA assays. An antibody can be said to competitively inhibit binding of the reference antibody to a given epitope by at least 90%, at least 80%, at least 70%, at least 60%, or at least 50%.

As used herein, the term "affinity" refers to a measure of the strength of the binding of an individual epitope with the CDR of an immunoglobulin molecule. See, *e.g.,* Harlow et al. (1988) Antibodies: A Laboratory Manual (Cold Spring Harbor Laboratory Press, 2nd ed.) pages 27-28 The term "sufficient affinity" as used herein, refers to a sufficient strength of binding of an anti-α-synuclein antibody or antigen binding fragment thereof to α-synuclein or an epitope thereof, to alter the net efflux of alpha synuclein from brain to blood, or from brain to CSF. As used herein, the term "net efflux" refers to the total flow of α-synuclein from brain to blood or brain to CSF.

As used herein, the term "avidity" refers to the overall stability of the complex between a population of immunoglobulins and an antigen, that is, the functional combining strength of an immunoglobulin mixture with the antigen. See, *e.g*., Harlow at pages 29-34. Avidity is related to both the affinity of individual immunoglobulin molecules in the population with specific epitopes, and also the valencies of the immunoglobulins and the antigen. For example, the interaction between a bivalent monoclonal antibody and an antigen with a highly repeating epitope structure, such as a polymer, would be one of high avidity.

Anti-α-synuclein antibodies or antigen-binding fragments, variants, or derivatives thereof as disclosed herein can also be described or specified in terms of their cross-reactivity. As used herein, the term "cross-reactivity" refers to the ability of an antibody, specific for one antigen, to react with a second antigen; a measure of relatedness between two different antigenic substances. Thus, an antibody is cross reactive if it binds to an epitope other than the one that induced its formation. The cross reactive epitope generally contains many of the same complementary structural features as the inducing epitope, and in some cases, can actually fit better than the original.

For example, certain antibodies have some degree of cross-reactivity, in that they bind related, but non-identical epitopes, e.g., epitopes with at least 95%, at least 90%, at least 85%, at least 80%, at least 75%, at least 70%, at least 65%, at least 60%, at least 55%, and at least 50% identity (as calculated using methods known in the art and described herein) to a reference epitope. An antibody can be said to have little or no cross-reactivity if it does not bind epitopes with less than 95%, less than 90%, less than 85%, less than 80%, less than 75%, less than 70%, less than 65%, less than 60%, less than 55%, and less than 50% identity (as calculated using methods known in the art and described herein) to a reference epitope. An antibody can be deemed "highly specific" for a certain epitope, if it does not bind any other analog, ortholog, or homolog of that epitope.

Anti-α-synuclein binding molecules, e.g., antibodies or antigen-binding fragments, variants or derivatives thereof, as described herein can also be described or specified in terms of their binding affinity to a polypeptide of the disclosure, e.g., human α-synuclein. Preferred binding affinities include those with a dissociation constant or Kd less than 5 x 10⁻² M, 10⁻² M, 5 x 10⁻³ M, 10⁻³ M, 5 x 10⁻⁴ M, 10⁻⁴ M, 5 x 10⁻⁵ M, 10⁻⁵ M, 5 x 10⁻⁶ M, 10⁻⁶ M, 5 x 10⁻⁷ M, 10⁻⁷ M, 5 x 10⁻⁸ M, 10⁻⁸ M, 5 x 10⁻⁹ M, 10⁻⁹ M, 5 x 10⁻¹⁰ M, 10⁻¹⁰ M, 5 x 10⁻¹¹ M, 10⁻¹¹ M, 5 x 10⁻¹² M, 10⁻¹² M, 5 x 10⁻¹³ M, 10⁻¹³ M, 5 x 10⁻¹⁴ M, 10⁻¹⁴ M, 5 x 10⁻¹⁵ M, or 10⁻¹⁵ M_{.}

Antibody fragments including single-chain antibodies can comprise the variable region(s) alone or in combination with the entirety or a portion of the following: hinge region, CH1, CH2, and CH3 domains. Also included are antigen-binding fragments also comprising any combination of variable region(s) with a hinge region, CH1, CH2, and CH3 domains. Binding molecules, e.g., antibodies, or antigen-binding fragments thereof disclosed herein can be from any animal origin including birds and mammals. The antibodies can be human, murine, donkey, rabbit, goat, guinea pig, camel, llama, horse, or chicken antibodies. In another embodiment, the variable region can be condricthoid in origin (e.g., from sharks).

As used herein, the term "chimeric antibody" will be held to mean any antibody wherein the immunoreactive region or site is obtained or derived from a first species and the constant region (which can be intact, partial or modified in accordance with the instant disclosure) is obtained from a second species. For example, the target binding region or site can be from a non-human source (e.g., mouse or primate) and the constant region can be human. Alternatively, a fully human binding region can be combined with a non-human (e.g., mouse) constant region.

As used herein, the term "murinized antibody" or "murinized immunoglobulin" refers to an antibody comprising one or more CDRs from a human antibody of the present disclosure; and a human framework region that contains amino acid substitutions and/or deletions and/or insertions that are based on a mouse antibody sequence. The human immunoglobulin providing the CDRs is called the "parent" or "acceptor" and the mouse antibody providing the framework changes is called the "donor". Constant regions need not be present, but if they are, they are usually substantially identical to mouse antibody constant regions, *i.e.* at least about 85- 90%, preferably about 95% or more identical. Hence, in some embodiments, a full length murinized human heavy or light chain immunoglobulin contains a mouse constant region, human CDRs, and a substantially human framework that has a number of "murinizing" amino acid substitutions. Typically, a "murinized antibody" is an antibody comprising a murinized variable light chain and/or a murinized variable heavy chain. For example, a murinized antibody would not encompass a typical chimeric antibody, e.g., because the entire variable region of a chimeric antibody is non-mouse. A modified antibody that has been "murinized" by the process of "murinization" binds to the same antigen as the parent antibody that provides the CDRs and is usually less immunogenic in mice, as compared to the parent antibody.

As used herein, the term "engineered antibody" refers to an antibody in which the variable domain in either the heavy or light chain or both is altered by at least partial replacement of one or more CDRs from an antibody of known specificity and, if necessary, by partial framework region replacement and sequence changing. Although the CDRs can be derived from an antibody of the same class or even subclass as the antibody from which the framework regions are derived, it is envisaged that the CDRs will be derived from an antibody of different class and preferably from an antibody from a different species. An engineered antibody in which one or more "donor" CDRs from a non-human antibody of known specificity is grafted into a human heavy or light chain framework region is referred to herein as a "humanized antibody." It can not be necessary to replace all of the CDRs with the complete CDRs from the donor variable domain to transfer the antigen binding capacity of one variable domain to another. Rather, it can only be necessary to transfer those residues that are necessary to maintain the activity of the target binding site.

As used herein, "human" or "fully human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described infra and, for example, in U.S. Pat. No. 5,939,598 by Kucherlapati et al. "Human" or "fully human" antibodies also include antibodies comprising at least the variable domain of a heavy chain, or at least the variable domains of a heavy chain and a light chain, where the variable domain(s) have the amino acid sequence of human immunoglobulin variable domain(s).

"Human" or "fully human" antibodies also include "human" or "fully human" antibodies, as described herein, that comprise, consist essentially of, or consist of, variants (including derivatives) of antibody molecules (*e.g*., the VH regions and/or VL regions) described herein, which antibodies or fragments thereof immunospecifically bind to an α-synuclein polypeptide or fragment or variant thereof. Standard techniques known to those of skill in the art can be used to introduce mutations in the nucleotide sequence encoding a human anti-α-synuclein antibody, including, but not limited to, site-directed mutagenesis and PCR-mediated mutagenesis which result in amino acid substitutions. Preferably, the variants (including derivatives) encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference VH region, VHCDR1, VHCDR2, VHCDR3, VL region, VLCDR1, VLCDR2, or VLCDR3.

In one aspect, the antibody of the disclosure is a human monoclonal antibody isolated from a human. Optionally, the framework region of the human antibody is aligned and adopted in accordance with the pertinent human germ line variable region sequences in the database; see, e.g., Vbase (http://vbase.mrc-cpe.cam.ac.uk/) hosted by the MRC Centre for Protein Engineering (Cambridge, UK). For example, amino acids considered to potentially deviate from the true germ line sequence could be due to the PCR primer sequences incorporated during the cloning process. Compared to artificially generated human-like antibodies such as single chain antibody fragments (scFvs) from a phage displayed antibody library or xenogeneic mice the human monoclonal antibody of the present disclosure is characterized by (i) being obtained using the human immune response rather than that of animal surrogates, i.e., the antibody has been generated in response to natural α-synuclein in its relevant conformation in the human body, (ii) having protected the individual or is at least significant for the presence of α-synuclein, and (iii) since the antibody is of human origin the risks of cross-reactivity against self-antigens is minimized. Thus, in accordance with the disclosure the terms "human monoclonal antibody", "human monoclonal autoantibody", "human antibody" and the like are used to denote an α-synuclein binding molecule which is of human origin, i.e. which has been isolated from a human cell such as a B cell or hybridoma thereof or the cDNA of which has been directly cloned from mRNA of a human cell, for example a human memory B cell. A human antibody is still "human" even if amino acid substitutions are made in the antibody, e.g., to improve binding characteristics.

Antibodies derived from human immunoglobulin libraries or from animals transgenic for one or more human immunoglobulins and that do not express endogenous immunoglobulins, as described *infra* and, for example in, US patent no 5,939,598 by Kucherlapati et al.*,* are denoted human-like antibodies in order distinguish them from truly human antibodies of the present disclosure.

As used herein, the term "sample" refers to any biological material obtained from a subject or patient. In one aspect, a sample can comprise blood, cerebrospinal fluid ("CSF"), or urine. In other aspects, a sample can comprise whole blood, plasma, B cells enriched from blood samples, and cultured cells *(e.g.,* B cells from a subject). A sample can also include a biopsy or tissue sample including neural tissue. In still other aspects, a sample can comprise whole cells and/or a lysate of the cells. Blood samples can be collected by methods known in the art. In one aspect, the pellet can be resuspended by vortexing at 4°C in 200 µl buffer (20 mM Tris, pH. 7.5, 0.5% Nonidet, 1 mM EDTA, 1 mM PMSF, 0.1M NaCl, IX Sigma Protease Inhibitor, and IX Sigma Phosphatase Inhibitors 1 and 2). The suspension can be kept on ice for 20 minutes with intermittent vortexing. After spinning at 15,000 x g for 5 minutes at about 4°C, aliquots of supernatant can be stored at about -70°C.

As used herein, the terms "treat" or "treatment" refer to both therapeutic treatment and prophylactic or preventative measures, wherein the object is to prevent or slow down (lessen) an undesired physiological change, infection, or disorder. Beneficial or desired clinical results include, but are not limited to, alleviation of symptoms, diminishment of extent of disease, stabilized *(i.e.,* not worsening) state of disease, clearance or reduction of an infectious agent in a subject, a delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the infection, condition, or disorder as well as those prone to have the condition or disorder or those in which the condition or disorder is to be prevented.

By "test subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, bears, and so on.

### II. TARGET POLYPEPTIDE DESCRIPTION

As used herein, the terms "α-synuclein", "alpha-synuclein", "a-synuclein" and "aSyn" are used interchangeably to specifically refer to the native monomer form of α-synuclein. The term "α-synuclein" is also used to generally identify other conformers of α-synuclein, for example, α-synuclein bonded to dopamine-quinone (DAQ) and oligomers or aggregates of α-synuclein. The term "α-synuclein" is also used to refer collectively to all types and forms of α-synuclein. The protein sequence for human α-synuclein is:

The amino acid sequence of α-synuclein can be retrieved from the literature and pertinent databases; see, *e.g.,* Ueda et al., PNAS 90: 1282-11286 (1993); GenBank swissprot: locus SYUA_HUMAN, accession number P37840. α-synuclein was originally identified in human brains as the precursor protein of the non-β-amyloid component of (NAC) of Alzheimer's disease (AD) plaques; *see, e.g.,* Ueda *et al., ibid.* α-synuclein, is a protein of 140 amino acids and exists in its native form as a random coil. However, changes in pH, molecular crowding, heavy metal content, and dopamine levels all affect protein conformation. Changes in conformation to oligomeric, proto-fibrillar, fibrillar, and aggregate moieties are thought to regulate protein toxicity. Increasing evidence indicates that dopamine-adducted α-synuclein has a faster time course to fibril formation compared to non-adducted protein. Furthermore, dopamine in the background of α-synuclein overexpression is toxic.

NAC, a highly hydrophobic domain within α-synuclein, is a peptide consisting of at least 28 amino acids residues (residues 60-87) and optionally 35 amino acid residues (residues 61-95). NAC displays a tendency to form a beta-sheet structure (Iwai et al., Biochemistry, 34: 10139-10145 (1995)). The amino acid sequences of NAC are described in Jensen et al., Biochem. J. 310: 91-94 (1995); GenBank accession number S56746 and Ueda *et al., ibid.*

Disaggregated α-synuclein or fragments thereof, including NAC, means monomeric peptide units. Disaggregated α-synuclein or fragments thereof are generally soluble, and are capable of self-aggregating to form soluble oligomers. Oligomers of α-synuclein and fragments thereof are usually soluble and exist predominantly as α-helices. Monomeric α-synuclein can be prepared *in vitro* by dissolving lyophilized peptide in neat DMSO with sonication. The resulting solution is centrifuged to remove any insoluble particulates. Aggregated α-synuclein or fragments thereof, including NAC, means oligomers of α-synuclein or fragments thereof which have associate into insoluble β-sheet assemblies. Aggregated α-synuclein or fragments thereof, including NAC, means also means fibrillar polymers. Fibrils are usually insoluble. Some antibodies bind either soluble α-synuclein or fragments thereof or aggregated α-synuclein or fragments thereof. Some antibodies bind to oligomers of α-synuclein more strongly than to monomeric forms or fibrillar forms. Some antibodies bind both soluble and aggregated α-synuclein or fragments thereof, and optionally oligomeric forms as well.

### III. ANTI-α-SYNUCLEIN ANTIBODIES

Antibodies that bind α-synuclein have been described in the art. See, for example, International Patent Publication WO 2010/069603.

The human anti-α-synuclein antibodies described herein specifically bind to α-synuclein and epitopes thereof and to various conformations of α-synuclein and epitopes thereof. For example, disclosed herein are antibodies that specifically bind α-synuclein, α-synuclein in its native monomer form, full-length and truncated α-synuclein and α-synuclein aggregates. For example, 12F4 antibody, as described herein, binds to full length α-synuclein and to α-synuclein truncations containing amino acids (aa) 1-60 in as tested by direct ELISA, pointing to an epitope of 12F4 within the N-terminal amphipathic repeat region of alpha synuclein. (*See* WO 2010/069603).

As used herein, reference to an antibody that "specifically binds", "selectively binds", or "preferentially binds" α-synuclein refers to an antibody that does not bind other unrelated proteins. In one example, an α-synuclein antibody disclosed herein can bind α-synuclein or an epitope thereof and show no binding above about 1.5 times background for other proteins. An antibody that "specifically binds" or "selectively binds" α-synuclein conformer refers to an antibody that does not bind all conformations of α-synuclein, *i.e.,* does not bind at least one other α-synuclein conformer. For example, disclosed herein are antibodies that can distinguish among monomeric and aggregated forms of α-synuclein, human and mouse α-synuclein; full-length α-synuclein and truncated forms as well as human α-synuclein versus β- and γ-synuclein. Since the human anti-α-synuclein antibodies of the present disclosure have been isolated from a pool of elderly subjects with no signs of Parkinsonism and exhibiting an α-synuclein-specific immune response the anti-α-synuclein antibodies disclosed herein can also be called "human auto-antibodies" in order to emphasize that those antibodies were indeed expressed by the subjects and have not been isolated from, for example a human immunoglobulin expressing phage library, which hitherto represented one common method for trying to provide human-like antibodies.

The disclosure generally relates to a method of diagnosing an elevated level of α-synuclein in the brain of a test subject, comprising administration of an antibody which specifically binds to α-synuclein, or an antigen-binding fragment, variant, or derivative thereof. Anti-α-synuclein antibodies can be used in the methods provided herein. Antibodies that can be used include, but are not limited to recombinant human α-synuclein antibodies NI-202.3G12, 12F4, or NI-202.3D8 and antigen-binding fragments, variants, or derivatives thereof which are fully described in International Patent Publication WO 2010/069603.

In certain embodiments, an anti-α-synuclein antibody or antigen-binding fragment, variant, or derivative thereof useful in the methods provided herein has an amino acid sequence that has at least about 80%, about 85%, about 88%, about 89%, about 90%, about 91%, about 92%, about 93%, about 94%, or about 95% sequence identity to the amino acid sequence for a reference anti-α-synuclein antibody molecule, for example those described herein. In a further embodiment, the binding molecule shares at least about 96%, about 97%, about 98%, about 99%, or 100% sequence identity to a reference antibody. In certain embodiments, the antibody or antigen-binding fragment thereof specifically binds to the same α-synuclein epitope as a reference antibody comprising an immunoglobulin heavy chain variable region (VH) and an immunoglobulin light chain variable region (VL), wherein the VH comprises amino acid sequence at least 80%, 85%, 90% 95% or 100% identical to SEQ ID NO: 2 and the VL comprises amino acid sequence at least 80%, 85%, 90% 95% or 100% identical to SEQ ID NO: 3, as shown in Table 2.

Further disclosed is the antibody or antigen-binding fragment, variant, or derivative thereof useful in the methods provided herein which specifically binds to the same α-synuclein epitope as a reference antibody comprising VH and a VL, wherein the VH comprises amino acid sequence identical to, or identical except for one, two, three, four, five, or more amino acid substitutions to SEQ ID NO: 2, and the VL comprises amino acid sequence identical to, or identical except for one, two, three, four, five, or more amino acid substitutions to SEQ ID NO: 3, as shown in Table 2.

Some embodiments include an anti-α-synuclein antibody or antigen-binding fragment, variant, or derivative thereof useful in the methods provided herein comprising a VH, where one or more of the VHCDR1, VHCDR2 or VHCDR3 regions of the VH are at least 80%, 85%, 90%, 95% or 100% identical to one or more reference heavy chain VHCDR1, VHCDR2 and/or VHCDR3 amino acid sequences of one or more of: SEQ ID NO: 4, SEQ ID NO: 5, SEQ ID NO: 6, as shown in Table 3.

Further disclosed is an anti-α-synuclein antibody or antigen-binding fragment, variant, or derivative thereof useful in the methods provided herein comprising a VH, where one or more of the VHCDR1, VHCDR2 or VHCDR3 regions of the VH are identical to, or identical except for four, three, two, or one amino acid substitutions, to one or more reference heavy chain VHCDR1, VHCDR2 or VHCDR3 amino acid sequences of one or more of: SEQ ID NO: 4, SEQ ID NO: 5, SEQ ID NO: 6, as shown in Table 3.

Also disclosed is an anti-α-synuclein antibody or antigen-binding fragment, variant, or derivative thereof useful in the methods provided herein comprising a VL, where one or more of the VLCDR1, VLCDR2 or VLCDR3 regions of the VL are at least 80%, 85%, 90%, 95% or 100% identical to one or more reference heavy chain VLCDR1, VLCDR2 or VLCDR3 amino acid sequences of one or more of: SEQ ID NO: 7, SEQ ID NO: 8, SEQ ID NO: 9, as shown in Table 3.

Some embodiments disclose an anti-α-synuclein antibody or antigen-binding fragment, variant, or derivative thereof useful in the methods provided herein comprising a VL, where one or more of the VLCDR1, VLCDR2 or VLCDR3 regions of the VL are identical to, or identical except for four, three, two, or one amino acid substitutions, to one or more reference heavy chain VLCDR1, VLCDR2 or VLCDR3 amino acid sequences of one or more of: SEQ ID NO: 7, SEQ ID NO: 8, SEQ ID NO: 9, as shown in Table 3.

In other embodiments, an anti-α-synuclein antibody or antigen-binding fragment, variant, or derivative thereof useful in the methods provided herein comprises, consists essentially of, or consists of VH and VL amino acid sequences at least 80%, 85%, 90% 95% or 100% identical to: SEQ ID NO: 2 and SEQ ID NO: 3, as shown in Table 2.

**Table 2: Reference VH and VL amino acid sequences***

| VH | VL |
|---|---|
| | |
| SEQ ID NO: 2 | SEQ ID NO: 3 |

| | |
|---|---|
| *VH and VL CDR1, CDR2, and CDR3 amino acid sequences are underlined | |

**Table 3: Reference VH and VL CDR1, CDR2, and CDR3 amino acid sequences**

| VHCDR1 | VHCDR2 | VHCDR3 | VLCDR1 | VLCDR2 | VLCDR3 |
|---|---|---|---|---|---|
| KAWMS SEQ ID NO: 4 | SEQ ID NO:5 | AH SEQ ID NO: 6 | SEQ ID NO: 7 | KDSERPS SEQ ID NO: 8 | QSPDSTNTYEV SEQ ID NO: 9 |

Also included for use in the methods described herein are polypeptides encoding anti-α-synuclein antibodies, or antigen-binding fragments, variants, or derivatives thereof as described herein, polynucleotides encoding such polypeptides, vectors comprising such polynucleotides, and host cells comprising such vectors or polynucleotides, all for producing anti-α-synuclein antibodies, or antigen-binding fragments, variants, or derivatives thereof for use in the methods described herein.

Suitable biologically active variants of anti-α-synuclein antibodies as described herein can be used in the methods of the disclosure. Such variants will retain the desired binding properties of the parent anti-α-synuclein antibody. Methods for making antibody variants are generally available in the art.

Methods for mutagenesis and nucleotide sequence alterations are well known in the art. See, for example, Walker and Gaastra, eds. (1983) Techniques in Molecular Biology (MacMillan Publishing Company, New York); Kunkel, Proc. Natl. Acad. Sci. USA 82:488-492 (1985); Kunkel et al., Methods Enzymol. 154:367-382 (1987); Sambrook et al. (1989) Molecular Cloning: A Laboratory Manual (Cold Spring Harbor, N.Y.); U.S. Pat. No. 4,873,192; and the references cited therein. Guidance as to appropriate amino acid substitutions that do not affect biological activity of the polypeptide of interest can be found in the model of Dayhoff et al. in Atlas of Protein Sequence and Structure (Natl. Biomed. Res. Found., Washington, D.C.), pp. 345-352 (1978). The model of Dayhoff *et al.* uses the Point Accepted Mutation (PAM) amino acid similarity matrix (PAM 250 matrix) to determine suitable conservative amino acid substitutions. Conservative substitutions, such as exchanging one amino acid with another having similar properties, can be preferred. Examples of conservative amino acid substitutions as taught by the PAM 250 matrix of the Dayhoff *et al.* model include, but are not limited to, Gly↔Ala, Val↔Ile↔Leu, Asp↔Glu, Lys↔Arg, Asn↔Gln, and Phe↔Trp↔Tyr.

Methods for measuring an anti-α-synuclein antibody or antigen-binding fragment, variant, or derivative thereof, binding specificity include, but are not limited to, standard competitive binding assays, assays for monitoring immunoglobulin secretion by T cells or B cells, T cell proliferation assays, apoptosis assays, ELISA assays, and the like. See, for example, such assays disclosed in WO 93/14125; Shi et al., Immunity 13:633-642 (2000); Kumanogoh et al., J Immunol 169:1175-1181 (2002); Watanabe et al., J Immunol 167:4321-4328 (2001); Wang et al., Blood 97:3498-3504 (2001); and Giraudon et al., J Immunol 172(2):1246-1255 (2004).

When discussed herein whether any particular polypeptide, including the constant regions, CDRs, VH domain or VL domains disclosed herein, is at least about 65%, about 70%, about 75%, about 80%, about 85%, about 90%, about 91%, about 92%, about 93%, about 94%, about 95%, about 96%, about 97%, about 98%, about 99%, or even about 100% identical to another polypeptide, the % identity can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, Wis. 53711). BESTFIT uses the local homology algorithm of Smith and Waterman (1981) Adv. Appl. Math. 2:482-489, to find the best segment of homology between two sequences. When using BESTFIT or any other sequence alignment program to determine whether a particular sequence is, for example, 95% identical to a reference sequence according to the present disclosure, the parameters are set, of course, such that the percentage of identity is calculated over the full length of the reference polypeptide sequence and that gaps in homology of up to 5% of the total number of amino acids in the reference sequence are allowed.

For purposes of the disclosure, percent sequence identity can be determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant can, for example, differ from a reference anti-α-synuclein antibody by as few as 1 to 15 amino acid residues, as few as 1 to 10 amino acid residues, such as 6-10, as few as 5, as few as 4, 3, 2, or even 1 amino acid residue. A "conservative amino acid substitution" is one in which the amino acid residue is replaced with an amino acid residue having a side chain with a similar charge. Families of amino acid residues having side chains with similar charges have been defined in the art. These families include amino acids with basic side chains (*e.g.,* lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (e.g., threonine, valine, isoleucine) and aromatic side chains (*e.g*., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity (*e.g*., the ability to bind an α-synuclein polypeptide).

For example, it is possible to introduce mutations only in framework regions or only in CDR regions of an antibody molecule. Introduced mutations can be silent or neutral missense mutations, *i.e.,* have no, or little, effect on an antibody's ability to bind antigen. These types of mutations can be useful to optimize codon usage, or improve a hybridoma's antibody production. Alternatively, non-neutral missense mutations can alter an antibody's ability to bind antigen. One of skill in the art would be able to design and test mutant molecules with desired properties such as no alteration in antigen binding activity or alteration in binding activity (e.g., improvements in antigen binding activity or change in antibody specificity). Following mutagenesis, the encoded protein can routinely be expressed and the functional and/or biological activity of the encoded protein, (e.g., ability to immunospecifically bind at least one epitope of an α-synuclein polypeptide) can be determined using techniques described herein or by routinely modifying techniques known in the art.

### IV. DIAGNOSING OR TRACKING METHODS USING ANTI-α-SYNUCLEIN ANTIBODIES

The present disclosure relates to the use of an anti-α-synuclein binding molecule, e.g., antibody or antigen-binding fragment thereof, for diagnosing an elevated level of α-synuclein in the brain of a test subject, i.e. human subject (e.g., for determining a subject's risk for developing a synucleinopathic disease), or for monitoring the progression of a synucleinopathic disease or a response to a synucleinopathic disease treatment in a test subject as characterized in the claims.

In certain embodiments, the methods as described herein are directed to the use of anti-α-synuclein antibodies, including antigen-binding fragments, variants, and derivatives thereof, described herein, to diagnose an elevated level of α-synuclein in the brain of a test subject by: (a) assaying the level of α-synuclein in a blood plasma, or a CSF sample obtained from the test subject at a specified interval following peripheral administration to the test subject of an anti-α-synuclein antibody or antigen-binding fragment thereof, wherein the anti-α-synuclein antibody or fragment thereof can bind α-synuclein with sufficient affinity to alter the net efflux of α-synuclein from brain to blood, or from brain to CSF; (b) comparing the assayed level of the α-synuclein in the test subject to a reference standard; wherein the difference or similarity between the level of α-synuclein in the plasma sample, or the CSF sample and the reference standard correlates with the level of α-synuclein in the brain of the test subject.

In other embodiments, the methods as described herein are directed to the use of anti-α-synuclein antibodies, including antigen-binding fragments, variants, and derivatives thereof, described herein, to diagnose an elevated level of α-synuclein in the brain of a test subject by: (a) assaying the level of α-synuclein in a blood plasma, or a CSF sample obtained from the test subject at a specified interval following peripheral administration to the test subject of an anti-α-synuclein antibody or antigen-binding fragment thereof, wherein the anti-α-synuclein antibody or fragment thereof stabilizes or sequesters α-synuclein in blood or CSF; (b) comparing the assayed level of the α-synuclein in the test subject to a reference standard; wherein the difference or similarity between the level of α-synuclein in the plasma sample, or the CSF sample and the reference standard correlates with the level of α-synuclein in the brain of the test subject.

In order to apply the methods and systems of the disclosure, samples from a patient can be obtained before or after the administration of an anti-α-synuclein antibody or antigen-binding fragment thereof. Samples can, for example, be requested by a healthcare provider (*e.g*., a doctor) or healthcare benefits provider, obtained and/or processed by the same or a different healthcare provider (*e.g*., a nurse, a hospital) or a clinical laboratory, and after processing, the results can be forwarded to yet another healthcare provider, healthcare benefits provider or the patient. Similarly, assaying the level of α-synuclein in the sample, comparing the assayed level of the α-synuclein in the test subject to the reference standard, evaluation of the results can be performed by one or more healthcare providers, healthcare benefits providers, and/or clinical laboratories.

As used herein, the term "healthcare provider" refers to individuals or institutions which directly interact and administer to living subjects, *e.g*., human patients. Non-limiting examples of healthcare providers include doctors, nurses, technicians, therapist, pharmacists, counselors, alternative medicine practitioners, medical facilities, doctor's offices, hospitals, emergency rooms, clinics, urgent care centers, alternative medicine clinics/facilities, and any other entity providing general and/or specialized treatment, assessment, maintenance, therapy, medication, and/or advice relating to all, or any portion of, a patient's state of health, including but not limited to general medical, specialized medical, surgical, and/or any other type of treatment, assessment, maintenance, therapy, medication and/or advice.

As used herein, the term "clinical laboratory" refers to a facility for the examination or processing of materials derived from a living subject, *e.g.,* a human being. Non-limiting examples of processing include biological, biochemical, serological, chemical, immunohematological, hematological, biophysical, cytological, pathological, genetic, or other examination of materials derived from the human body for the purpose of providing information, *e.g*., for the diagnosis, prevention, or treatment of any disease or impairment of, or the assessment of the health of living subjects, *e.g*., human beings. These examinations can also include procedures to collect or otherwise obtain a sample, prepare, determine, measure, or otherwise describe the presence or absence of various substances in the body of a living subject, *e.g.,* a human being, or a sample obtained from the body of a living subject, *e.g.,* a human being. In certain aspects a clinical laboratory can be "centralized" or "local", meaning that a small number or a single laboratory makes all measurements of samples submitted from all outside sources. In other aspects, multiple clinical laboratories, also referred to as "satellite" or "global" laboratories, can be validated to all provide standard, reliable results that can be easily compared.

As used herein, the term "healthcare benefits provider" encompasses individual parties, organizations, or groups providing, presenting, offering, paying for in whole or in part, or being otherwise associated with giving a patient access to one or more healthcare benefits, benefit plans, health insurance, and/or healthcare expense account programs.

In some aspects, a healthcare provider can administer or instruct another healthcare provider to administer an anti-α-synuclein antibody or antigen-binding fragment thereof. A healthcare provider can implement or instruct another healthcare provider or patient to perform the following actions: obtain a sample, process a sample, submit a sample, receive a sample, transfer a sample, analyze or measure a sample, quantify a sample, provide the results obtained after analyzing/measuring/quantifying a sample, receive the results obtained after analyzing/measuring/quantifying a sample, compare/score the results obtained after analyzing/measuring/quantifying one or more samples, provide the comparison/score from one or more samples, obtain the comparison/score from one or more samples, administer a therapy or therapeutic agent (*e.g.,* an anti-α-synuclein antibody or antigen-binding fragment thereof), commence the administration of a therapy, cease the administration of a therapy, continue the administration of a therapy, temporarily interrupt the administration of a therapy, increase the amount of an administered therapeutic agent, decrease the amount of an administered therapeutic agent, continue the administration of an amount of a therapeutic agent, increase the frequency of administration of a therapeutic agent, decrease the frequency of administration of a therapeutic agent, maintain the same dosing frequency on a therapeutic agent, replace a therapy or therapeutic agent by at least another therapy or therapeutic agent, combine a therapy or therapeutic agent with at least another therapy or additional therapeutic agent.

In some aspects, a healthcare benefits provider can authorize or deny, for example, collection of a sample, processing of a sample, submission of a sample, receipt of a sample, transfer of a sample, analysis or measurement a sample, quantification a sample, provision of results obtained after analyzing/measuring/quantifying a sample, transfer of results obtained after analyzing/measuring/quantifying a sample, comparison/scoring of results obtained after analyzing/measuring/quantifying one or more samples, transfer of the comparison/score from one or more samples, administration of a therapy or therapeutic agent, commencement of the administration of a therapy or therapeutic agent, cessation of the administration of a therapy or therapeutic agent, continuation of the administration of a therapy or therapeutic agent, temporary interruption of the administration of a therapy or therapeutic agent, increase of the amount of administered therapeutic agent, decrease of the amount of administered therapeutic agent, continuation of the administration of an amount of a therapeutic agent, increase in the frequency of administration of a therapeutic agent, decrease in the frequency of administration of a therapeutic agent, maintain the same dosing frequency on a therapeutic agent, replace a therapy or therapeutic agent by at least another therapy or therapeutic agent, or combine a therapy or therapeutic agent with at least another therapy or additional therapeutic agent.

In addition a healthcare benefits providers can, e.g., authorize or deny the prescription of a therapy, authorize or deny coverage for therapy, authorize or deny reimbursement for the cost of therapy, determine or deny eligibility for therapy, etc.

In some aspects, a clinical laboratory can, for example, collect or obtain a sample, process a sample, submit a sample, receive a sample, transfer a sample, analyze or measure a sample, quantify a sample, provide the results obtained after analyzing/measuring/quantifying a sample, receive the results obtained after analyzing/measuring/quantifying a sample, compare/score the results obtained after analyzing/measuring/quantifying one or more samples, provide the comparison/score from one or more samples, obtain the comparison/score from one or more samples,

The above enumerated actions can be performed by a healthcare provider, healthcare benefits provider, or patient automatically using a computer-implemented method (e.g., via a web service or stand-alone computer system).

In some embodiments the methods as described herein are directed to the use of anti-α-synuclein antibodies, including antigen-binding fragments, variants, and derivatives thereof, described herein, to diagnose an elevated level of α-synuclein in the brain of a test subject by: (a) peripherally administering an anti-α-synuclein antibody or antigen-binding fragment thereof to the test subject, wherein the antibody or fragment thereof can bind α-synuclein with sufficient affinity to alter the net efflux of the α-synuclein from brain to blood, or from brain to CSF; (b) obtaining a blood plasma sample, or a CSF sample from the test subject at a specified time interval following administration, and submitting the plasma sample, or the CSF sample for determination of the level of the α-synuclein; (c) comparing the level of the α-synuclein in blood plasma sample to a reference standard; wherein the difference or similarity between the level of the α-synuclein in the plasma sample, or the CSF sample and the reference standard correlates with level of the α-synuclein in the brain of the test subject.

In other embodiments the methods as described herein are directed to the use of anti-α-synuclein antibodies, including antigen-binding fragments, variants, and derivatives thereof, described herein, to diagnose an elevated level of α-synuclein in the brain of a test subject by: (a) peripherally administering an anti-α-synuclein antibody or antigen-binding fragment thereof to the test subject, wherein the antibody or fragment thereof stabilizes or sequesters α-synuclein in blood or CSF; (b) obtaining a blood plasma sample, or a CSF sample from the test subject at a specified time interval following administration, and submitting the plasma sample, or the CSF sample for determination of the level of the α-synuclein; (c) comparing the level of the α-synuclein in blood plasma sample to a reference standard; wherein the difference or similarity between the level of the α-synuclein in the plasma sample, or the CSF sample and the reference standard correlates with level of the α-synuclein in the brain of the test subject. In some embodiments, anti-α-synuclein antibodies, including antigen-binding fragments, variants, and derivatives thereof, described herein, can bind α-synuclein with sufficient affinity to alter the net efflux of the α-synuclein from brain to blood, or from brain to CSF and stabilize or sequester α-synuclein in blood or CSF.

The test subject to be diagnosed can be asymptomatic or preclinical for the disease. In specific embodiments test subjects include individuals who are pre-symptomatic or have preclinical synucleopathic disease.

In specific embodiments, the "reference standard" in the method described herein comprises measured levels of α-synuclein in one or more control subjects, wherein the control subjects include normal healthy individuals, and individuals with synucleinopathies of varying severity. For example, the control subject has a synucleinopathic disease, for example Parkinson's disease (PD), dementia with Lewy bodies (DLB) or the Lewy body variant of Alzheimer's disease (LBVAD), wherein a similarity between the level of α-synuclein and the reference standard indicates that the subject to be diagnosed has a synucleinopathic disease. Alternatively, or in addition as a second control the control subject does not have a synucleinopathic disease, wherein a difference between the level of α-synuclein and the reference standard indicates that the subject to be diagnosed has a synucleinopathic disease. Preferably, the subject to be diagnosed and the control subject(s) are age-matched.

In some embodiments the methods as described herein, further comprise comparing the level of the α-synuclein in the plasma sample *(i.e.,* test sample) to a plasma sample *(i.e.,* baseline sample) obtained from the test subject prior to administration of the anti-α-synuclein antibody including antigen-binding fragments, variants, and derivatives thereof. In other embodiments the methods as described herein, further comprise comparing the level of the α-synuclein in the CSF sample *(i.e.,* test sample) to a CSF sample *(i.e.,* baseline sample) obtained from the test subject prior to administration of the anti-α-synuclein antibody including antigen-binding fragments, variants, and derivatives thereof. For example, the comparison can be made to a baseline sample instead of or in addition to comparison with a reference standard. In this respect, the baseline sample can be used to calibrate the test samples to the reference standard (*e.g*., the measurement is a difference or a ratio rather than an absolute value).

By a further embodiment, the anti-α-synuclein binding molecules, in particular anti-α-synuclein antibodies, as described herein, can also be used in a method for the diagnosis of a disorder in an individual by obtaining a body fluid sample from the tested individual which may be a blood sample, a lymph sample, a CSF sample, or any other body fluid sample, and contacting the body fluid sample with an anti-α-synuclein antibody as described herein, under conditions enabling the formation of antibody-antigen complexes. The level of such complexes is then determined by methods known in the art, a level significantly higher than that formed in a control sample indicating the disease in the tested individual. In the same manner, the specific antigen bound by the anti-α-synuclein antibodies as described herein can also be used. Thus, the disclosure relates to *an in vitro* immunoassay comprising an anti-α-synuclein binding molecule, *e.g*., antibody or antigen-binding fragment thereof of the disclosure.

The level of α-synuclein can be assessed by any suitable method known in the art comprising, e.g., analyzing α-synuclein by one or more techniques chosen from Western blot, immunoprecipitation, enzyme-linked immunosorbent assay (ELISA), radioimmunoassay (RIA), fluorescent activated cell sorting (FACS), two-dimensional gel electrophoresis, mass spectroscopy (MS), matrix-assisted laser desorption/ionization-time of flight-MS (MALDI-TOF), surface-enhanced laser desorption ionization-time of flight (SELDI-TOF), high performance liquid chromatography (HPLC), fast protein liquid chromatography (FPLC), multidimensional liquid chromatography (LC) followed by tandem mass spectrometry (MS/MS), and laser densitometry. Preferably, said in vivo imaging of α-synuclein comprises positron emission tomography (PET), single photon emission tomography (SPECT), near infrared (NIR) optical imaging or magnetic resonance imaging (MRI).

As is well known in the medical arts, dosages for any one patient depends upon many factors, including the patient's size, body surface area, age, the particular compound to be administered, sex, time and route of administration, general health, and other drugs being administered concurrently. Generally, the dosage can range, *e.g.,* from about 0.0001 to 100 mg/kg, and more usually 0.01 to 5 mg/kg (*e.g.,* 0.02 mg/kg, 0.25 mg/kg, 0.5 mg/kg, 0.75 mg/kg, 1 mg/kg, 2 mg/kg, etc.), of the host body weight. For example dosages can be 1 mg/kg body weight or 10 mg/kg body weight or within the range of 1-10 mg/kg. Doses intermediate in the above ranges are also intended to be within the scope of the disclosure. The term "peripheral administration" is described elsewhere herein.

In certain embodiments, an antibody-based array can be used, which is for example loaded with anti-α-synuclein antibodies or equivalent antigen-binding molecules of the disclosure which specifically recognize α-synuclein. Design of microarray immunoassays is summarized in Kusnezow et al., Mol. Cell Proteomics 5:1681-1696 (2006).

In some embodiments, the methods as described herein are also directed to the use of anti-α-synuclein antibodies, including antigen-binding fragments, variants, and derivatives thereof, to track the α-synuclein level in the brain of a subject being treated for a synucleinopathic disease, comprising assaying the level of α-synuclein in the subject's blood plasma, or the subject's CSF at a specified time following peripheral administration of an anti-α-synuclein antibody or antigen-binding fragment thereof, wherein the antibody or fragment thereof can bind α-synuclein with sufficient affinity to alter the net efflux of the α-synuclein from brain to blood, or brain to CSF; and wherein the α-synuclein level in the subject's blood plasma, or the subject's CSF correlates with the level in the subject's brain. In specific embodiments, the method as described herein, further comprises assaying the level of α-synuclein in the subject's blood plasma, or the subject's CSF at a specified time following additional peripheral administrations of the anti-α-synuclein antibody or antigen-binding fragment thereof, thereby plotting the change in the α-synuclein level in the subject's brain over time.

In some embodiments, the methods as described herein are also directed to the use of anti-α-synuclein antibodies, including antigen-binding fragments, variants, and derivatives thereof, to track the α-synuclein level in the brain of a subject being treated for a synucleinopathic disease, comprising assaying the level of α-synuclein in the subject's blood plasma, or the subject's CSF at a specified time following peripheral administration of an anti-α-synuclein antibody or antigen-binding fragment thereof, wherein the antibody or fragment thereof stabilizes or sequesters α-synuclein in blood or CSF; and wherein the α-synuclein level in the subject's blood plasma, or the subject's CSF correlates with the level in the subject's brain. In specific embodiments, the method as described herein, further comprises assaying the level of α-synuclein in the subject's blood plasma, or the subject's CSF at a specified time following additional peripheral administrations of the anti-α-synuclein antibody or antigen-binding fragment thereof, thereby plotting the change in the α-synuclein level in the subject's brain over time.

Some embodiments include methods as described herein, where the specified time interval is less than 12 months, less than 11 months, less than 10 months, less than 9 months, less than 8 months, less than 7 months, less than 6 months, less than 5 months, less than 4 months, less than 3 months, less than 2 months, less than a month, less than a week, or less than or equal to 24 hours, or less than or equal to 3 hours.

### V. COMPOSITIONS AND ADMINISTRATION METHODS

The methods of preparing and administering anti-α-synuclein antibodies, or antigen-binding fragments, variants, or derivatives thereof to a subject in need thereof are well known to or are readily determined by those skilled in the art. The route of *administration* of an anti-α-synuclein antibody, or antigen-binding fragment, variant, or derivative thereof, can be, for example, oral, parenteral, by inhalation or topical. The term "peripheral administration" as used herein includes, *e.g*., intravenous, intraarterial, intraperitoneal, intramuscular, subcutaneous, rectal, or vaginal administration. While all these forms of administration are clearly contemplated as being within the scope of the disclosure, an example of a form for administration would be a solution for injection, in particular for intravenous or intraarterial injection or drip. A suitable pharmaceutical composition for injection can comprise a buffer (*e.g.* acetate, phosphate or citrate buffer), a surfactant (*e.g.* polysorbate), optionally a stabilizer agent (*e.g*. human albumin), etc.

As discussed herein, anti-α-synuclein antibodies, or antigen-binding fragments, variants, or derivatives thereof can be formulated so as to facilitate administration and promote stability of the active agent. In certain embodiments, pharmaceutical compositions in accordance with the present disclosure comprise a pharmaceutically acceptable, non-toxic, sterile carrier such as physiological saline, non-toxic buffers, preservatives and the like. For the purposes of the instant application, a pharmaceutically effective amount of an anti-α-synuclein antibody, or antigen-binding fragment, variant, or derivative thereof, shall be held to mean an amount sufficient to achieve effective binding to a target and to achieve a benefit, *e.g.,* to alter the net efflux of α-synuclein from brain to blood, or to alter the net efflux of α-synuclein from brain to CSF.

The pharmaceutical compositions used in this disclosure comprise pharmaceutically acceptable carriers, including, *e.g*., ion exchangers, alumina, aluminum stearate, lecithin, *serum* proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

Preparations for peripheral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include, *e.g*., water, alcoholic/aqueous solutions, emulsions or suspensions, including saline and buffered media. In the subject disclosure, pharmaceutically acceptable carriers include, but are not limited to, 0.01-0.1 M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives can also be present such as, for example, antimicrobials, antioxidants, chelating agents, and inert gases and the like.

More particularly, pharmaceutical compositions suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, for example, water, ethanol, polyol (*e.g.,* glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Suitable formulations for use in the therapeutic methods disclosed herein are described in Remington's Pharmaceutical Sciences (Mack Publishing Co.) 16th ed. (1980).

Prevention of the action of microorganisms can be achieved by various antibacterial and antifungal agents, for example, parabens, chlorobutanol, phenol, ascorbic acid, thimerosal and the like. In many cases, it will be preferable to include isotonic agents, for example, sugars, polyalcohols, such as mannitol, sorbitol, or sodium chloride in the composition. Prolonged absorption of the injectable compositions can be brought about by including in the composition an agent which delays absorption, for example, aluminum monostearate and gelatin.

In any case, sterile injectable solutions can be prepared by incorporating an active compound (e.g., an anti-α-antibody, or antigen-binding fragment, variant, or derivative thereof, by itself or in combination with other active agents) in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yields a powder of an active ingredient plus any additional desired ingredient from a previously sterile-filtered solution thereof. The preparations for injections are processed, filled into containers such as ampoules, bags, bottles, syringes or vials, and sealed under aseptic conditions according to methods known in the art. Further, the preparations can be packaged and sold in the form of a kit. Such articles of manufacture can have labels or package inserts indicating that the associated compositions are useful for treating a subject suffering from, or predisposed to a disease or disorder.

Parenteral formulations can be a single bolus dose, an infusion or a loading bolus dose followed with a maintenance dose. These compositions can be administered at specific fixed or variable intervals, e.g., once a day, or on an "as needed" basis.

Certain pharmaceutical compositions, as disclosed herein, can be orally administered in an acceptable dosage form including, e.g., capsules, tablets, aqueous suspensions or solutions. Certain pharmaceutical compositions also can be administered by nasal aerosol or inhalation. Such compositions can be prepared as solutions in saline, employing benzyl alcohol or other suitable preservatives, absorption promoters to enhance bioavailability, and/or other conventional solubilizing or dispersing agents.

The amount of an anti-α-synuclein antibody, or fragment, variant, or derivative thereof, to be combined with the carrier materials to produce a single dosage form will vary depending upon the host treated and the particular mode of administration. The composition can be administered as a single dose, multiple doses or over an established period of time in an infusion. Dosage regimens also can be adjusted to provide the optimum desired response *(e.g.,* a therapeutic or prophylactic response).

The practice of the disclosure will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis et al. U.S. Pat. No: 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Caner and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Maryland (1989).

General principles of antibody engineering are set forth in Antibody Engineering, 2nd edition, C.A.K. Borrebaeck, Ed., Oxford Univ. Press (1995). General principles of protein engineering are set forth in Proteins Engineering, A Practical Approach, Rickwood, D., et al., Eds., IRL Press at Oxford Univ. Press, Oxford, Eng. (1995). General principles of antibodies and antibody-hapten binding are set forth in: Nisonoff, A., Molecular Immunology, 2nd ed., Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (eds), Basic and Clinical -Immunology (8th ed.), Appleton & Lange, Norwalk, CT (1994) and Mishell and Shiigi (eds), Selected Methods in Cellular Immunology, W.H. Freeman and Co., New York (1980).

Standard reference works setting forth general principles of immunology include Current Protocols in Immunology, John Wiley & Sons, New York; Klein, J., Immunology: The Science of Self-Nonself Discrimination, John Wiley & Sons, New York (1982); Kennett, R., et al., eds., Monoclonal Antibodies, Hybridoma: A New Dimension in Biological Analyses, Plenum Press, New York (1980); Campbell, A., "Monoclonal Antibody Technology" in Burden, R., et al., eds., Laboratory Techniques in Biochemistry and Molecular Biology, Vol. 13, Elsevere, Amsterdam (1984), Kuby Immunnology 4th ed. Ed. Richard A. Goldsby, Thomas J. Kindt and Barbara A. Osborne, H. Freemand & Co. (2000); Roitt, I., Brostoff, J. and Male D., Immunology 6th ed. London: Mosby (2001); Abbas A., Abul, A. and Lichtman, A., Cellular and Molecular Immunology Ed. 5, Elsevier Health Sciences Division (2005); Kontermann and Dubel, Antibody Engineering, Springer Verlan (2001); Sambrook and Russell, Molecular Cloning: A Laboratory Manual. Cold Spring Harbor Press (2001); Lewin, Genes VIII, Prentice Hall (2003); Harlow and Lane, Antibodies: A Laboratory Manual, Cold Spring Harbor Press (1988); Dieffenbach and Dveksler, PCR Primer Cold Spring Harbor Press (2003).

### EXAMPLES

Detailed descriptions of conventional methods, such as those employed herein can be found in the cited literature. Unless indicated otherwise below, identification of α-synuclein-specific B cells and molecular cloning of α-synuclein antibodies displaying specificity of interest as well as their recombinant expression and functional characterization has been or can be performed as described in the Examples and Supplementary Methods section of international applications PCT/EP2008/000053 published as WO2008/081008, and international applications PCT/EP2009/009186 published as WO2010/069603.

### Example 1: Dose dependent human α-synuclein plasma spike upon 12F4 administration in transgenic mice overexpressing human α-synuclein

This example describes determination of human α-synuclein levels in mouse plasma in transgenic mice overexpressing human α-synuclein after injection of 12F4 antibody. Three and half months old transgenic mice overexpressing human wild-type (wt) α-synuclein (PDGFβ-h[wt] α-synuclein; i.e., D-line; Masliah et al., Science, 287(5456):1265-9 (2000) were intraperitoneally injected with a single dose of 0, 0.3, 1, 3, 10 or 30 mg/kg 12F4 antibody. Functional recombinant monoclonal antibodies 12F4 and chimeric 12F4 were obtained upon co-transfection into CHO cells (or any other appropriate recipient cell line of human or mouse origin) of an Ig-heavy-chain expression vector and a kappa or lambda Ig-light-chain expression vector. Recombinant monoclonal antibody was subsequently purified from the conditioned medium using a standard Protein A column purification as described in WO2008/081008. Recombinant human monoclonal antibody can be produced in unlimited quantities using either transiently or stably transfected cells. Chimeric 12F4 antibody had primer induced mutations at the N-termini of the Ig-variable regions being adjusted to the germ line (GL) sequences of human variable heavy and light chains (*see* WO2010/069603), and was expressed as a chimeric molecule where the adjusted human variable domains were fused to mouse IgG2a constant regions.

D-line transgenic α-synuclein mice were kept under standard housing conditions on a reversed 12h:12h light/dark cycle with free access to food and water. The treatment groups were balanced for age and gender. 24 hrs after the injection, plasma samples were prepared and plasma concentrations of human α-synuclein were determined by a sandwich ELISA (Invitrogen, USA). Plasma samples were diluted 1:4 and standard was prepared in dilution buffer with 1:4 plasma of wt mice. Results were controlled for influence of 12F4 antibody on ELISA readings. 12F4 antibody plasma levels were determined by a human Fcg capture ELISA using recombinant 12F4 of known concentration as standard. Standard was prepared in PBS containing diluted plasma from the wild-type mice.

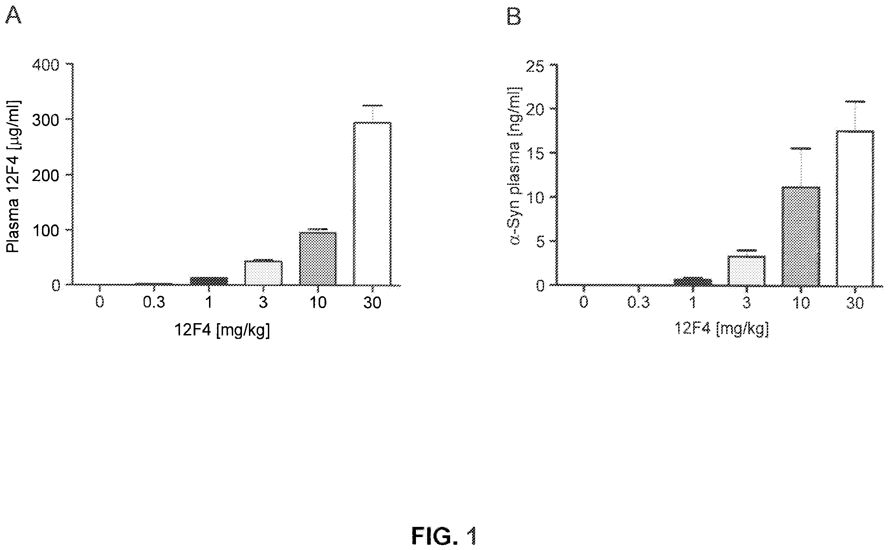

Plasma levels of human α-synuclein were significantly increased after a single dose of 1, 3, 10 or 30 mg/kg 12F4 antibody when compared to vehicle control. The increase in plasma human α-synuclein is significantly dose dependent (Figures 1 A-B). No significant levels of human α-synuclein were detected in mice that were treated with vehicle only (Figure 1B).

### Example 2: Time course of α-synuclein plasma spike and 12F4 plasma concentration

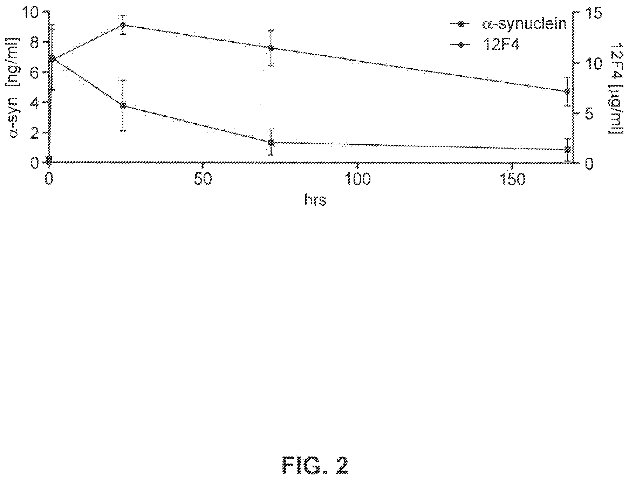

This example describes the time course of changes in human α-synuclein levels and 12F4 antibody levels in mouse plasma in transgenic mice overexpressing human α-synuclein, measured over time. Eight month old transgenic mice overexpressing human α-synuclein A53T (Prp-h[A53T] α-synuclein] *(*Giasson et al., Neuron, 34: 521-533 (2001)) were intraperitoneally injected with a single dose of 5 mg/kg 12F4 antibody and plasma samples were collected at time points 0, 1, 24, 72 and 168 hrs post injection. Plasma human α-synuclein was quantified by ELISA as described in Example 1. Figure 2 shows that plasma human α-synuclein peaks already in 1 hr time point and then declines over time. On the other hand, highest levels of 12F4 antibody were found at 24 hrs time point and 12F4 plasma levels appeared to decline more slowly than human α-synuclein levels.

### Example 3: Acute high dose 12F4 treatment of transgenic mice overexpressing human α-synuclein reduces brain human α-synuclein levels that correlate with plasma human α-synuclein levels

This example describes the determination of human α-synuclein levels in brain samples after injection with 12F4 antibody. Three and half months old transgenic mice overexpressing human wild-type α-synuclein (PDGFβ-h[wt] α-synuclein; D-line] were intraperitoneally injected with four 50 mg/kg doses of 12F4 antibody within 8 days (72, 144 and 192 hrs post first injection). 24 hrs after the last injection animals were sacrificed and perfused with PBS. Cortex and hippocampus were homogenized in PBS and soluble (PBS-soluble) and insoluble (PBS-insoluble) brain fractions were prepared by differential centrifugation. Specifically, brains were removed, dissected and frozen at -80°C. Frozen brain tissues were homogenized in 10 volumes (v/w) of PBS using a dounce homogenizer (500 rpm, 30 strokes) and sonification for 1 min. Cell debris was removed by centrifugation at 5000 g for 5 min (4°C). Supernatant (SN) was centrifuged for 1 hr (4°C) at 35000 rpm (Ti51 rotor; Beckman-Coulter). Resulting SN was designated soluble fraction. The pellet was resuspended in 1% Trition PBS and sonicated (3x 1min). This fraction was designated insoluble fraction.

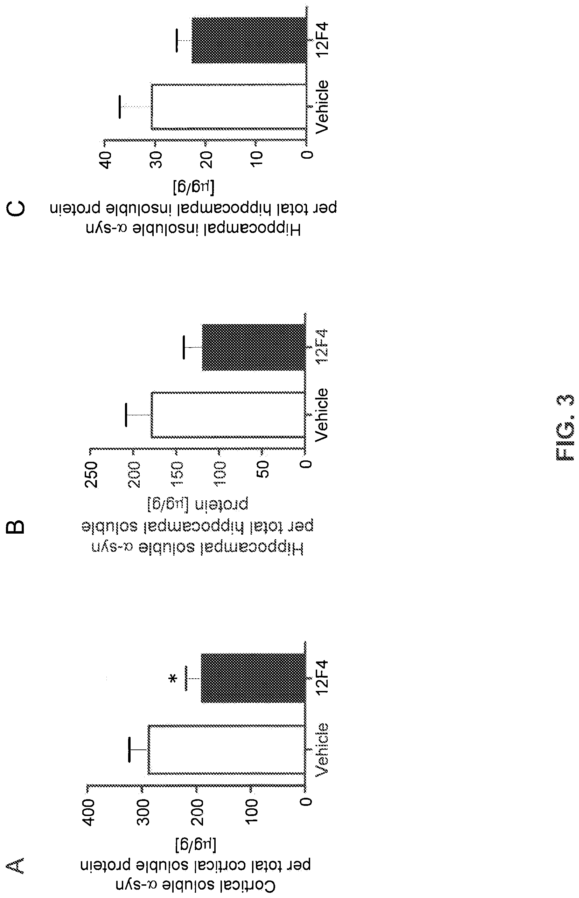

Human α-synuclein levels in both fractions were quantified by a sandwich ELISA (Invitrogen, USA) and normalized to protein content. As shown in Figure 3A cortical soluble human α-synuclein levels of 12F4 treated mice were significantly reduced by 34% (190 ± 29 µg/g for 12F4 vs. 288 ± 36 µg/g for vehicle control, n=10, p<0.05, Student's Test). Similarly, a 33% reduction of soluble hippocampal human α-synuclein (Figure 3B)-(119 ± 22 µg/g for 12F4 vs. 178 ± 30 µg/g for vehicle control, n=9-10, p=0.14, Student's Test) and a 26% reduction of insoluble hippocampal human α-synuclein (Figure 3C)-(23 ± 3 µg/g for 12F4 vs. 31 ± 6 µg/g for vehicle control, n=9-10, p=0.29, Student's Test) after acute 12F4 treatment was observed. These results show that a short treatment with 12F4 leads to a reduction of brain α-synuclein pathology in transgenic mice overexpressing human α-synuclein.

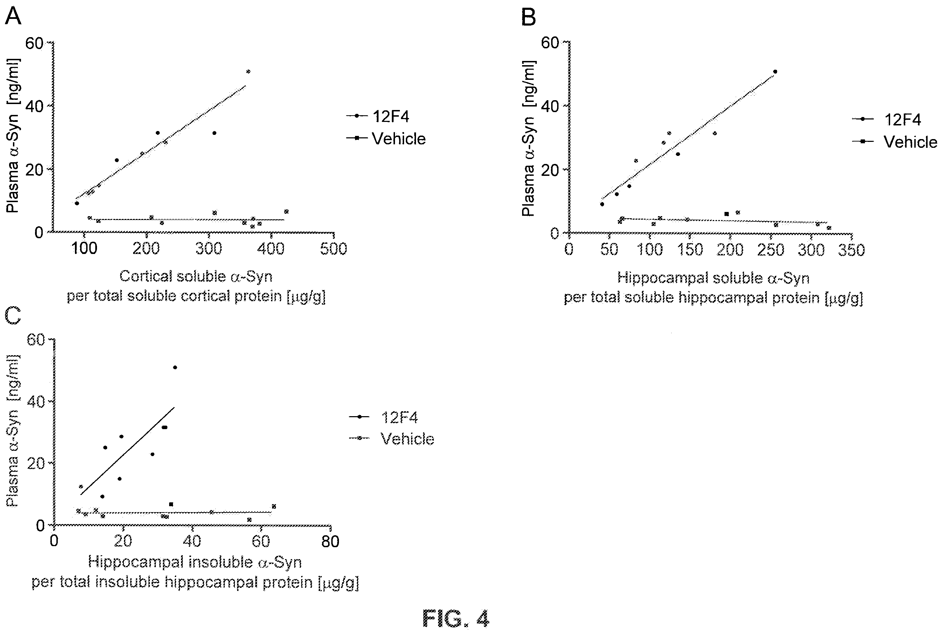

In order to see if the observed human α-synuclein plasma rise was linked to brain α-synuclein pathology, plasma human α-synuclein levels were plotted against brain human α-synuclein levels. There was a highly significant correlation between plasma human α-synuclein levels and soluble cortical human α-synuclein levels (Figure 4A), with soluble hippocampal human α-synuclein levels (Figure 4B), and insoluble hippocampal human α-synuclein levels (Figure 4C) after acute 12F4 treatment. No correlation between plasma and brain α-synuclein was observed upon vehicle treatment.

### Example 4: Correlation between plasma and brain human α-synuclein levels after chronic 12F4 treatment of transgenic mice overexpressing human α-synuclein

This example describes the determination of human α-synuclein levels in brain samples after weekly injections with 12F4 antibody for six months.

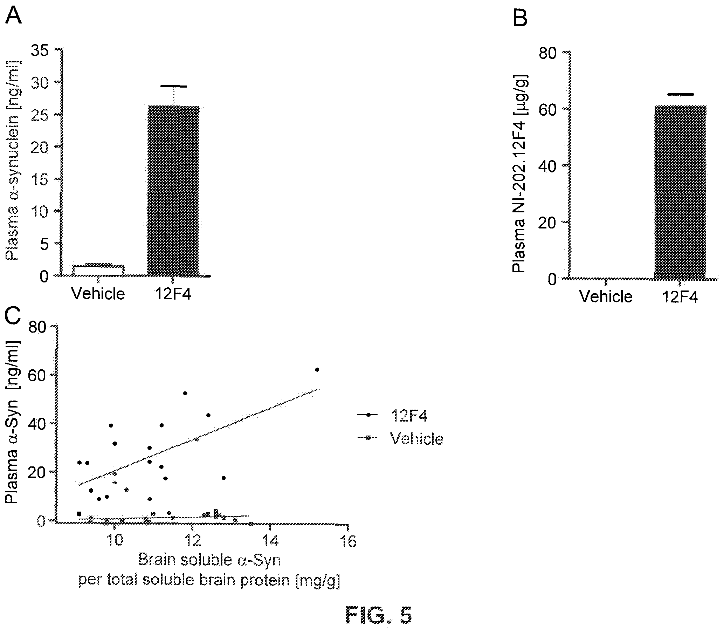

Six month old transgenic mice overexpressing human wt α-synuclein A30P (Thyl-h[A30P]-α-synuclein) (Kahle et al., Am J Pathol., 159(6): 2215-2225 (2001)) were intraperitoneally injected weekly with 10 mg/kg chimeric 12F4 for 6 months. Plasma and brain samples were prepared 24 hrs after last injection. Cortex/hippocampus were homogenized together in PBS and soluble (PBS-soluble) and insoluble (PBS-insoluble) brains fractions were prepared by differential centrifugation as described in Example 3. Human α-synuclein levels in both fractions were quantified by ELISA and normalized to protein content as described in Example 3. Plasma human α-synuclein levels (Figure 5A) and chimeric 12F4 levels (Figure 5B) were determined by ELISA. Plasma human α-synuclein levels were determined as described in Example 1, and chimeric 12F4 plasma levels were determined using a direct α-synuclein ELISA using recombinant chimeric 12F4 of known concentration as standard.

In order to see if plasma and brain α-synuclein correlate upon chronic treatment with chimeric 12F4, plasma human α-synuclein levels were plotted against human brain α-synuclein levels. There was a significant correlation between plasma and brain α-synuclein levels after chronic treatment for six month with chimeric 12F4 of transgenic mice overexpressing human α-synuclein. (Figure 5C).

### Example 5: α-synuclein cerebrospinal fluid (CSF) spike upon 12F4 administration in cynomolgus monkeys

This example describes determination of 12F4 levels in serum and cerebrospinal fluid (CSF), as well as endogenous α-synuclein levels in CSF of cynomolgus monkeys upon 12F4 administration. Three male naïve cynomolgus monkeys were intravenously injected with a single dose of 10 mg/kg 12F4. Animals were fasted 1 to 12 minutes prior to the 12F4 administration and for the 2 hours post-dose CSF sample collection. Blood samples (approximately 0.5 ml/sample) were collected from femoral vein/artery at 0.5, 2, 5, 24, 48, 72, 96, 168, 240, 336, 408, 504, 672 and 840 hours post-dose. Samples were allowed to clot for at least 30 minutes and centrifuged under ambient conditions following completion of the sample collection at each intervall. The resulting serum was separated and stored fozen at -50 to -90° until shipped on dry ice for analysis. 12F4 serum levels were determined by a sandwich ELISA (Covance).

12F4 and endogenous α-synuclein concentrations were also determined by ELISA (Covance) in the CSF samples collected from the cisterna magna at various time points post-dose. Prior to the CSF sample collection animals were sedated with an intramuscular (IM) injection of 0.1 mg/kg acepromazine maleate, with additional maintenance doses as necessary. Anesthesia was induced with an IM injection of 20 mg/kg Ketamine. The back of the head was shaved for access to the cisterna magna and the access site was prepared with chlorhexidine scrub and chlorhexidine solution within a sterile field. The animal was placed in a lateral recumbent position and the head was brought forward until the chin rested on the chest. Using aseptic technique, an over the needle catheter was used to access the cisterna magna. The animals were administered 0.01 mg/kg buprenorphine IM three times daily, approximately every 6 to 9 hours (beginning prior to CSF collection) on each day of the sample collection. Following anesthesia and CSF sampling, the animals were closely monitored during recovery for physiological disturbances including cardiovascular/respiratory depression, hypothermia, and excessive bleeding from the surgical site. The CSF samples (approximately 0.2 mL/sample) were collected at the 2, 24, 72, 168, 336, 504 and 672 hours post-dose from the cisterna magna and placed on ice. The samples were stored frozen at -50 to -90°C until shipped on dry ice for analysis.

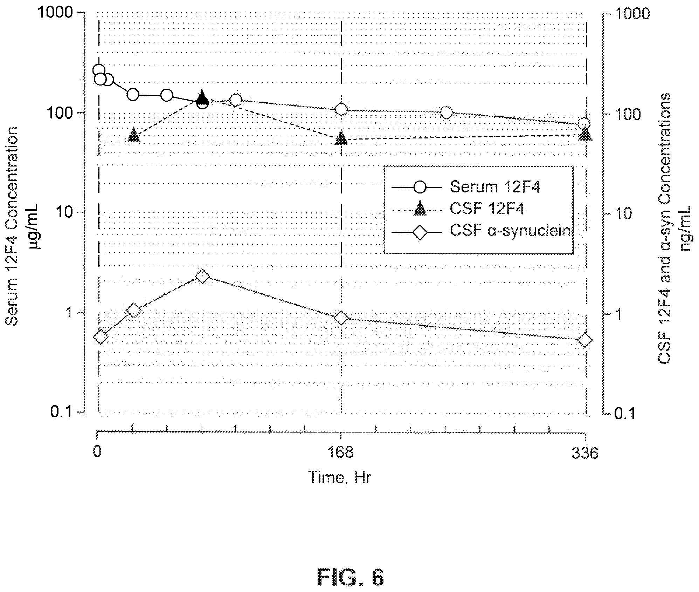

CSF/serum 12F4 ratio was around 0.1 % as expected for a human IgG antibody. CSF α-synuclein increased about 5-fold upon 12F4 treatment. (Figure 6).

### Example 6: In vivo microdialysis in transgenic α-synuclein mice

This example describes determination of α-synuclein levels in plasma and brain interstitial fluid (ISF) upon 12F4 administration in transgenic α-synuclein A53T mice. *In vivo* microdialysis in transgenic α-synuclein mouse ISF was performed upon administration of 12F4 or vehicle control (control IgG antibody). Specifically, guide cannulas were stereotaxically implanted in the striatum of 6-9 months old A53T α-synuclein transgenic mice (B6;C3-Tg(PrP-SNCA*A53T)83Vle/J) under isoflurane anesthesia (4-2.5%). The head was shaved and the skin was cut with a sterile scalpel to expose the skull. Bore holes were made above the right striatum according to the atlas of Paxinos and Franklin (The Mouse Brain in Stereotaxic Coordinates, Second Edition (2004)) (coordinates, AP = +0.5 mm, ML = -2.2 mm, DV = -2.4 mm). CMA-12 guide cannulas (CMA Microdialysis AB, Sweden) were inserted and fixed to the skull with stainless steel screws and dental cement. Mice were removed from the stereotaxic device and allowed to recover in individual cages. Five days after surgery, mice were removed to the microdialysis cage(s). CMA-12 custom made probes (2 mm, 100 kDa cut-off) were inserted and connected to a CMA pump with a constant flow rate of 0.6 µl/min. Perfusion was performed in artificial CSF containing BSA as an osmotic agent. Prior to sample collection, the probe was allowed to equilibrate for 4-20 hours with the same flow rate. Baseline samples were collected mostly bihourly for 2 hrs. 12F4 or vehicle control, were intraperitoneally injected in a single dose of 30 mg/kg. Upon injection samples were collected hourly for approximately 24 hours. All samples were collected using a refrigerated fraction collector and stored at -80°C until analyzed by an in house ultra-sensitive α-synuclein sandwich ELISA *(*Emmanouilidou et al., PLoS ONE 6(7): e22225 (2011)). Plasma samples were also collected pre-dose and at 2 and 24 hours post-dose, and then were analyzed by an human α-synuclein specific sandwich ELISA (Invitrogen, Carlsbad CA).

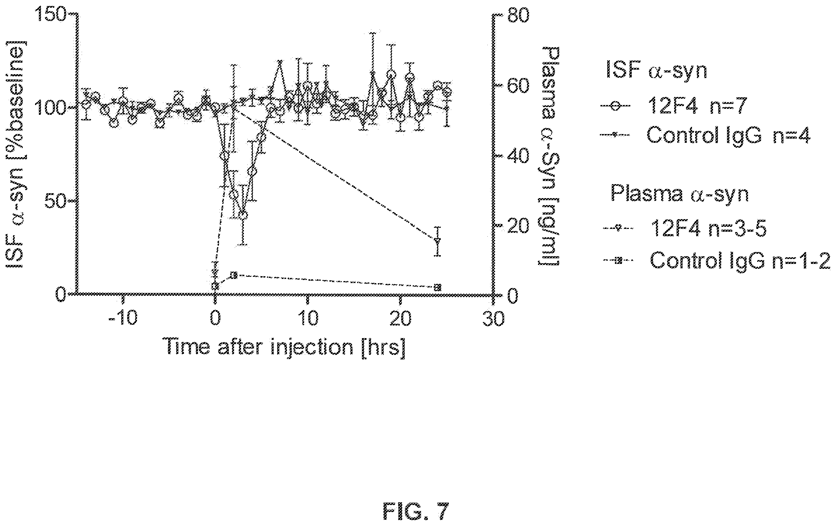

There was an approximately 60% reduction in extracellular, ISF α-synuclein, 2-3 hours post 12F4 administration. Vehicle control did not alter ISF α-synuclein levels in the microdialysate. (Figure 7).

### Example 7: Effect of anti-α-synuclein antibody on cerebrospinal fluid (CSF) α-synuclein concentration in an AAV-α-synuclein rat model

The effect of anti-α-synuclein antibody on CSF α-synuclein concentration will be evaluated in an Adeno-associated viral (AAV)-α-synuclein rat model. AAV vectors will be created that express either the wild-type human α-synuclein or one of the human α-synuclein sequence variants that are associated with familial Parkinson's disease (*e.g*., A53T or A30P). The AAV-α-synuclein vector will be injected into a specific region of the adult rat brain (*e.g*., striatum, cortex or hippocampus) or into the lateral ventricle of a neonatal rat. A period of one to several months will be allowed for the concentration of human α-synuclein to build up in the brain. At that time, rats will be treated with an anti-α-synuclein antibody by intraperitoneal or intravenous administration and samples of CSF will be taken at various times. The concentration of α-synuclein will be measured in the CSF samples by ELISA.

## Claims

1. An in vitro method of diagnosing an elevated level of α-synuclein in the brain of a human subject comprising:
(a) assaying the level of α-synuclein in a blood plasma sample or cerebrospinal fluid (CSF) sample obtained from the human subject at a specified time interval following peripheral administration to the human subject of an anti-α-synuclein antibody or antigen-binding fragment thereof, wherein the antibody or fragment thereof can bind α-synuclein with sufficient affinity to alter the net efflux of α-synuclein from brain to blood or CSF;
(b) comparing the assayed level of the α-synuclein in the blood plasma sample or CSF sample to a reference standard; wherein the difference or similarity between the level of α-synuclein in the blood plasma sample or CSF sample and the reference standard correlates with the level of α-synuclein in the brain of the human subject.

2. The method of claim 1, further comprising comparing the level of the α-synuclein in the blood plasma sample to a blood plasma sample obtained from the human subject prior to administration of the anti-α-synucleinantibody or antigen-binding fragment thereof, or comparing the level of the α-synuclein in the CSF sample to a CSF sample obtained from the human subject prior to administration of the anti-α-synuclein antibody or antigen-binding fragment thereof.

3. The method of claim 1 or 2, wherein the reference standard comprises measured levels of α-synuclein in one or more control subjects, wherein the control subjects are normal healthy individuals, or individuals with synucleinopathies of varying severity.

4. An in vitro method of tracking the α-synuclein level in the brain of a human subject being treated for a synucleinopathic disease, comprising assaying the level of α-synuclein in a sample of the human subject's blood plasma or CSF at a specified time interval following peripheral administration of an anti-α-synuclein antibody or antigen-binding fragment thereof, wherein the antibody or fragment thereof can bind α-synuclein with sufficient affinity to alter the net efflux of the α-synuclein from brain to blood_or CSF; and wherein the α-synuclein level in the human subject's blood plasma or CSF correlates with the level in the human subject's brain.

5. The method of claim 4, further comprising assaying the level of α-synuclein in a sample of the human subject's blood plasma or CSF at a specified time following additional peripheral administrations of the anti-α-synuclein antibody or antigen-binding fragment thereof, thereby plotting the change in the α-synuclein level in the human subject's brain over time.

6. The method of any one of claims 1 to 5, wherein the antibody or antigen-binding fragment thereof specifically binds to the same α-synuclein epitope as reference antibody comprising a VH and a VL, wherein the VH comprises SEQ ID NO: 2 and the VL comprises SEQ ID NO: 3.

7. The method of any one of claims 1 to 6, wherein the antibody or antigen-binding fragment thereof competitively inhibits reference antibody comprising a VH and a VL, wherein the VH comprises SEQ ID NO: 2 and the VL comprises SEQ ID NO: 3 from binding to α-synuclein.

8. The method of any one of claims 1 to 5, wherein the antibody or antigen-binding fragment thereof comprises a heavy chain variable region (VH) and a light chain variable region (VL), and wherein the VH and/or VL comprise one or more of the following sequences:
(a) the VH comprises a complementarity determining region-1 (VHCDR1) amino acid sequence of SEQ ID NO: 4;
(b) the VH comprises a complementarity determining region-2 (VHCDR2) amino acid sequence of SEQ ID NO: 5;
(c) the VH comprises a complementarity determining region-3 (VHCDR3) amino acid sequence of SEQ ID NO: 6;
(d) the VL comprises a complementarity determining region-1 (VLCDR1) amino acid sequence of SEQ ID NO: 7;
(e) the VL comprises a complementarity determining region-2 (VLCDR2) amino acid sequence of SEQ ID NO: 8; and/or
(f) the VL comprises a complementarity determining region-3 (VLCDR3) amino acid sequence of SEQ ID NO: 9.

9. The method of any one of claims 1 to 5, wherein the antibody or antigen-binding fragment thereof comprises a VH and a VL, wherein the VH comprises VHCDR1, VHCDR2, and VHCDR3 amino acid sequences of SEQ ID NOs: 4, 5, 6, and wherein the VL comprises VLCDR1, VLCDR2, and VLCDR3 amino acid sequences of SEQ ID NOs: 7, 8, 9.

10. The method of any one of claims 1 to 5, wherein the antibody or antigen binding fragment thereof comprises a VH amino acid sequence of SEQ ID NO: 2 and a VL amino acid sequence of SEQ ID NO: 3.

11. The method of any one of claims 1 to 10, wherein the antibody or antigen binding fragment thereof is a single chain Fv fragment (scFv), an F(ab') fragment, an F(ab) fragment, or an F(ab')₂ fragment.

12. The method of any one of claims 1 to 11, wherein the administration has been by intravenous injection of the antibody or antigen binding fragment thereof.

13. The method of any one of claims 1 to 12, wherein the antibody or antigen binding fragment thereof is human.

14. The method of any one of claims 1 to 13, wherein the specified time interval is less than a week.

15. The method of any one of claims 4, 5 or 6 to 14 as far as dependent from claim 4 or 5, wherein the synucleinopathic disease is selected from the group consisting of Parkinson's disease (PD), Parkinson's disease dementia (PDD), dementia with Lewy bodies (DLB), the Lewy body variant of Alzheimer's disease (LBVAD), multiple systems atrophy (MSA), pure autonomic failure (PAF), neurodegeneration with brain iron accumulation type-1 (NBIA-I), Alzheimer's disease, Pick disease, juvenile-onset generalized neuroaxonal dystrophy (Hallervorden-Spatz disease), amyotrophic lateral sclerosis, traumatic brain injury, and Down syndrome.

## Patentansprüche

1. In-vitro-Verfahren zum Diagnostizieren einer hohen Konzentration von α-Synuclein im Gehirn eines menschlichen Individuums, umfassend:
(a) Untersuchen der Konzentration von α-Synuclein in einer Blutplasmaprobe oder Cerebrospinalflüssigkeits(CSF)-Probe, die von dem menschlichen Individuum in einem spezifizierten Zeitintervall im Anschluss an eine periphere Verabreichung eines Anti-α-Synuclein-Antikörpers oder eines Antigen-bindenden Fragments davon an das menschliche Individuum erhalten wird, wobei der Antikörper oder das Fragment davon α-Synuclein mit ausreichend Affinität binden kann, um den Netto-Efflux von α-Synuclein von dem Gehirn in das Blut oder die CSF zu verändern;
(b) Vergleichen der untersuchten Konzentration des α-Synucleins in der Blutplasmaprobe oder CSF-Probe mit einem Referenzstandard; wobei die Differenz oder Ähnlichkeit zwischen der Konzentration von α-Synuclein in der Blutplasmaprobe oder CSF-Probe und dem Referenzstandard mit der Konzentration von α-Synuclein im Gehirn des menschlichen Individuums korreliert.

2. Verfahren nach Anspruch 1, ferner umfassend das Vergleichen der Konzentration des α-Synucleins in der Blutplasmaprobe mit einer Blutplasmaprobe, die von dem menschlichen Individuum vor der Verabreichung des Anti-α-Synuclein-Antikörpers oder des Antigen-bindenden Fragments davon erhalten wird, oder das Vergleichen der Konzentration des α-Synucleins in der CSF-Probe mit einer CSF-Probe, die von dem menschlichen Individuum vor der Verabreichung des Anti-α-Synuclein-Antikörpers oder des Antigen-bindenden Fragments davon erhalten wird.

3. Verfahren nach Anspruch 1 oder 2, wobei der Referenzstandard gemessene Konzentrationen von α-Synuclein in einem oder mehreren Kontrollindividuen umfasst, wobei die Kontrollindividuen normale gesunde Individuen oder Individuen mit Synucleinopathien mit unterschiedlicher Schwere sind.

4. In-vitro-Verfahren zum Verfolgen der α-Synuclein-Konzentration im Gehirn eines menschlichen Individuums, das wegen einer synucleinopathischen Erkrankung behandelt wird, umfassend das Untersuchen der Konzentration von α-Synuclein in einer Probe des Blutplasmas oder der CSF des menschlichen Individuums in einem spezifizierten Zeitintervall im Anschluss an eine periphere Verabreichung eines Anti-α-Synuclein-Antikörpers oder eines Antigen-bindenden Fragments davon, wobei der Antikörper oder das Fragment davon α-Synuclein mit ausreichend Affinität binden kann, um den Netto-Efflux des α-Synucleins von dem Gehirn in das Blut oder die CSF zu verändern; und wobei die α-Synuclein-Konzentration in dem Blutplasma oder der CSF des menschlichen Individuums mit der Konzentration im Gehirn des menschlichen Individuums korreliert.

5. Verfahren nach Anspruch 4, ferner umfassend das Untersuchen der Konzentration von α-Synuclein in einer Probe des Blutplasmas oder der CSF des menschlichen Individuums zu einem spezifizierten Zeitpunkt im Anschluss an zusätzliche periphere Verabreichungen des Anti-α-Synuclein-Antikörpers oder des Antigen-bindenden Fragments davon, wodurch die Änderung der α-Synuclein-Konzentration im Gehirn des menschlichen Individuums zeitlich dargestellt wird.

6. Verfahren nach einem der Ansprüche 1 bis 5, wobei der Antikörper oder das Antigen-bindende Fragment davon spezifisch an das gleiche α-Synuclein-Epitop bindet wie der Referenzantikörper, umfassend eine VH und eine VL, wobei die VH SEQ ID NO: 2 umfasst und die VL SEQ ID NO: 3 umfasst.

7. Verfahren nach einem beliebigen der Ansprüche 1 bis 6, wobei der Antikörper oder das Antigen-bindende Fragment davon den Referenzantikörper, umfassend eine VH und eine VL, wobei die VH SEQ ID NO: 2 umfasst und die VL SEQ ID NO: 3 umfasst, im Hinblick auf die Bindung an α-Synuclein kompetitiv hemmt.

8. Verfahren nach einem beliebigen der Ansprüche 1 bis 5, wobei der Antikörper oder das Antigen-bindende Fragment davon eine variable Region der schweren Kette (VH) und eine variable Region der leichten Kette (VL) umfasst, und wobei die VH und/oder VL eine oder mehrere der folgenden Sequenzen umfasst bzw. umfassen:
(a) die VH umfasst eine Aminosäuresequenz der komplementaritätsbestimmenden Region-1 (VHCDR1) von SEQ ID NO: 4;
(b) die VH umfasst eine Aminosäuresequenz der komplementaritätsbestimmenden Region-2 (VHCDR2) von SEQ ID NO: 5;
(c) die VH umfasst eine Aminosäuresequenz der komplementaritätsbestimmenden Region-3 (VHCDR3) von SEQ ID NO: 6;
(d) die VL umfasst eine Aminosäuresequenz der komplementaritätsbestimmenden Region-1 (VLCDR1) von SEQ ID NO: 7;
(e) die VL umfasst eine Aminosäuresequenz der komplementaritätsbestimmenden Region-2 (VLCDR2) von SEQ ID NO: 8; und/oder
(f) die VL umfasst eine Aminosäuresequenz der komplementaritätsbestimmenden Region-3 (VLCDR3) von SEQ ID NO: 9.

9. Verfahren nach einem der Ansprüche 1 bis 5, wobei der Antikörper oder das Antigen-bindende Fragment davon eine VH und eine VL umfasst, wobei die VH Aminosäuresequenzen der VHCDR1, VHCDR2 und VHCDR3 von SEQ ID NO: 4, 5, 6 umfasst, und wobei die VL Aminosäuresequenzen der VLCDR1, VLCDR2 und VLCDR3 von SEQ ID NO: 7, 8, 9 umfasst.

10. Verfahren nach einem der Ansprüche 1 bis 5, wobei der Antikörper oder das Antigen-bindende Fragment davon eine VH-Aminosäuresequenz von SEQ ID NO: 2 und eine VL-Aminosäuresequenz von SEQ ID NO: 3 umfasst.

11. Verfahren nach einem der Ansprüche 1 bis 10, wobei der Antikörper oder das Antigen-bindende Fragment davon ein einkettiges Fv-Fragment (scFv), ein F(ab')-Fragment, ein F(ab)-Fragment oder ein F(ab')₂-Fragment ist.

12. Verfahren nach einem der Ansprüche 1 bis 11, wobei die Verabreichung durch eine intravenöse Injektion des Antikörpers oder des Antigen-bindenden Fragments davon erfolgt ist.

13. Verfahren nach einem der Ansprüche 1 bis 12, wobei der Antikörper oder das Antigen-bindende Fragment davon menschlich ist.

14. Verfahren nach einem der Ansprüche 1 bis 13, wobei das spezifizierte Zeitintervall weniger als einer Woche entspricht.

15. Verfahren nach einem der Ansprüche 4, 5 oder 6 bis 14, sofern abhängig von Anspruch 4 oder 5, wobei die synucleinopathische Erkrankung ausgewählt ist aus der Gruppe, bestehend aus Parkinson (PD), Parkinson-Demenz (PDD), Demenz mit Lewy-Körperchen (DLB), Lewy-Körperchen-Variante der Alzheimer (LBVAD), multiple Systematrophie (MSA), reine Dysautonomie (PAF), Neurodegeneration mit Eisenablagerung im Gehirn Typ 1 (NBIA-I), Alzheimer, Pick-Krankheit, generalisierte juvenile neuroaxonale Dystrophie (Hallervorden-Spatz-Krankheit), amyotrophe Lateralsklerose, Schädel-Hirn-Trauma und Down-Syndrom.

## Revendications

1. Procédé in vitro de diagnostic d'un niveau élevé d'α-synucléine dans le cerveau d'un sujet humain comprenant :
(a) le dosage du niveau d'α-synucléine dans un échantillon de plasma sanguin ou un échantillon de liquide céphalo-rachidien (LCR) obtenu à partir du sujet humain à un intervalle de temps spécifié après l'administration périphérique au sujet humain d'un anticorps anti α-synucléine ou d'un fragment de liaison à un antigène de celui-ci, dans lequel l'anticorps ou un fragment de celui-ci peut lier une α-synucléine avec une affinité suffisante pour modifier l'efflux net d'α-synucléine du cerveau au sang ou au LCR ;
(b) la comparaison du niveau analysé de l'α-synucléine dans l'échantillon de plasma sanguin ou l'échantillon de LCR à un indice de référence ; dans lequel la différence ou la similitude entre le niveau d'α-synucléine dans l'échantillon de plasma sanguin ou l'échantillon de LCR et l'indice de référence est en corrélation avec le niveau d'α-synucléine dans le cerveau du sujet humain.

2. Procédé selon la revendication 1, comprenant en outre la comparaison du niveau de l'α-synucléine dans l'échantillon de plasma sanguin avec un échantillon de plasma sanguin obtenu à partir du sujet humain avant l'administration de l'anticorps anti α-synucléine ou d'un fragment de liaison à un antigène de celui-ci, ou la comparaison du niveau de l'α-synucléine dans l'échantillon de LCR à un échantillon de LCR obtenu à partir du sujet humain avant l'administration de l'anticorps anti α-synucléine ou d'un fragment de liaison à un antigène de celui-ci.

3. Procédé selon la revendication 1 ou 2, dans lequel l'indice de référence comprend des niveaux mesurés d'α-synucléine chez un ou plusieurs sujets témoins, dans lequel les sujets témoins sont des individus sains normaux, ou des individus présentant des synucléinopathies de gravité variable.

4. Procédé in vitro de suivi du niveau d'α-synucléine dans le cerveau d'un sujet humain traité pour une maladie synucléinopathique, comprenant l'analyse du niveau d'α-synucléine dans un échantillon de plasma sanguin ou de LCR du sujet humain à un intervalle de temps spécifié après l'administration périphérique d'un anticorps anti α-synucléine ou d'un fragment de liaison à un antigène de celui-ci, dans lequel l'anticorps ou un fragment de celui-ci peut lier une α-synucléine avec une affinité suffisante pour modifier l'efflux net de l'α-synucléine du cerveau au sang ou au LCR ; et dans lequel le niveau d'α-synucléine dans le plasma sanguin ou LCR du sujet humain est en corrélation avec le niveau dans le cerveau du sujet humain.

5. Procédé selon la revendication 4, comprenant en outre l'analyse du niveau d'α-synucléine dans un échantillon du plasma sanguin ou du LCR du sujet humain à un moment spécifié après des administrations périphériques supplémentaires de l'anticorps anti α-synucléine ou d'un fragment de liaison à un antigène de celui-ci, traçant ainsi le changement du niveau d'α-synucléine dans le cerveau du sujet humain au fil du temps.

6. Procédé selon l'une quelconque des revendications 1 à 5, dans lequel l'anticorps ou un fragment de liaison à un antigène de celui-ci se lie spécifiquement au même épitope d'α-synucléine que l'anticorps de référence comprenant une VH et une VL, dans lequel la VH comprend SEQ ID NO : 2 et la VL comprend SEQ ID NO : 3.

7. Procédé selon l'une quelconque des revendications 1 à 6, dans lequel l'anticorps ou un fragment de liaison à un antigène de celui-ci inhibe de manière compétitive l'anticorps de référence comprenant une VH et une VL, dans lequel la VH comprend SEQ ID NO : 2 et la VL comprend SEQ ID NO : 3 à partir de la liaison à une α-synucléine.

8. Procédé selon l'une quelconque des revendications 1 à 5, dans lequel l'anticorps ou un fragment de liaison à un antigène de celui-ci comprend une région variable de chaîne lourde (VH) et une région variable de chaîne légère (VL), et dans lequel la VH et/ou VL comprennent une ou plusieurs des séquences suivantes :
(a) la VH comprend une séquence d'acides aminés de la région-1 (VHCDR1) déterminante de complémentarité de SEQ ID NO : 4 ;
(b) la VH comprend une séquence d'acides aminés de la région-2 (VHCDR2) déterminante de complémentarité de SEQ ID NO : 5 ;
(c) la VH comprend une séquence d'acides aminés de la région-3 (VHCDR3) déterminante de complémentarité de SEQ ID NO : 6 ;
(d) la VL comprend une séquence d'acides aminés de la région-1 (VLCDR1) déterminante de complémentarité de SEQ ID NO : 7 ;
(e) la VL comprend une séquence d'acides aminés de la région-2 (VLCDR2) déterminante de complémentarité de SEQ ID NO : 8 ; et/ou
(f) la VL comprend une séquence d'acides aminés de la région-3 (VLCDR3) déterminante de complémentarité de SEQ ID NO : 9.

9. Procédé selon l'une quelconque des revendications 1 à 5, dans lequel l'anticorps ou un fragment de liaison à un antigène de celui-ci comprend une VH et une VL, dans lequel la VH comprend des séquences d'acides aminés VHCDR1, VHCDR2 et VHCDR3 de SEQ ID NO : 4, 5, 6, et dans lequel la VL comprend des séquences d'acides aminés VLCDR1, VLCDR2 et VLCDR3 de SEQ ID NO : 7, 8, 9.

10. Procédé selon l'une quelconque des revendications 1 à 5, dans lequel l'anticorps ou un fragment de liaison à un antigène de celui-ci comprend une séquence d'acides aminés VH de SEQ ID NO : 2 et une séquence d'acides aminés VL de SEQ ID NO : 3.

11. Procédé selon l'une quelconque des revendications 1 à 10, dans lequel l'anticorps ou un fragment de liaison à un antigène de celui-ci est un fragment Fv à chaîne unique (scFv), un fragment F(ab'), un fragment F(ab) ou un fragment F(ab')₂.

12. Procédé selon l'une quelconque des revendications 1 à 11, dans lequel l'administration a été réalisée par injection intraveineuse de l'anticorps ou d'un fragment de liaison à un antigène de celui-ci.

13. Procédé selon l'une quelconque des revendications 1 à 12, dans lequel l'anticorps ou un fragment de liaison à un antigène de celui-ci est humain.

14. Procédé selon l'une quelconque des revendications 1 à 13, dans lequel l'intervalle de temps spécifié est inférieur à une semaine.

15. Procédé selon l'une quelconque des revendications 4, 5 ou 6 à 14 dans la mesure où il dépend de la revendication 4 ou 5, dans lequel la maladie synucléinopathique est choisie dans le groupe constitué de la maladie de Parkinson (PD), la démence de la maladie de Parkinson (PDD), la démence à corps de Lewy (DLB), la variante à corps de Lewy de la maladie d'Alzheimer (LBVAD), l'atrophie de systèmes multiples (MSA), l'insuffisance autonome pure (PAF), la neurodégénerescence avec accumulation de fer dans le cerveau de type 1 (NBIA-I), la maladie d'Alzheimer, la maladie de Pick, la dystrophie neuroaxonale généralisée à apparition juvénile (maladie de Hallervorden-Spatz), la sclérose latérale amyotrophique, la lésion cérébrale traumatique et le syndrome de Down.
